# EUROPEAN PATENT SPECIFICATION

(11) **EP 3 572 509 B1**
(45) Date of publication and mention of the grant of the patent: **20.09.2023**
(21) Application number: 18741479.2
(22) Date of filing: 18.01.2018
(51) Int. Cl.: C12Q 1/689, C12N 15/09, C12N 1/20, C12Q 1/04

(54) **PRIMER SET, PROBE, KIT, AND METHOD FOR DETECTING MYCOBACTERIUM KANSASII**
PRIMERSET, SONDE, KIT UND VERFAHREN ZUM NACHWEIS DES MYCOBACTERIUMS KANSASII
ENSEMBLE D'AMORCES, SONDE, KIT ET PROCÉDÉ DE DÉTECTION DE MYCOBACTERIUM KANSASII

(30) Priority: 19.01.2017 JP 2017007871
(43) Date of publication of application: 27.11.2019
(73) Proprietor: Kaneka Corporation, Osaka-shi, Osaka 530-8288 (JP)
(72) Inventor: MIYAMOTO, Shigehiko, Takasago-shi Hyogo 676-8688 (JP); SANO, Sotaro, Takasago-shi Hyogo 676-8688 (JP); TOMONO, Jun, Takasago-shi Hyogo 676-8688 (JP); JIKIHARA, Takaaki, Takasago-shi Hyogo 676-8688 (JP); HATA, Hiroyuki, Tokyo 103-0024 (JP); KAWAI, Akiko, Tokyo 103-0024 (JP)
(74) Representative: Vossius & Partner Patentanwälte Rechtsanwälte mbB
(86) International application number: PCT/JP2018/001325
(87) International publication number: WO 2018/135560

(56) References cited:
- WO-A1-2006/121134
- WO-A2-98/36089
- WO-A2-2009/017902
- JP-A- H0 630 796
- JP-A- H1 057 098
- JP-A- H11 155 589
- JP-A- 2001 128 679
- JP-A- 2009 039 103
- AKAMATU, NORIHIKO: "Rapid and accurate detection of mycobacterium kansasii in sputum by second-step PCR following COBAS AMPLICOR PCR", The Journal of the Japanese Society for Clinical Microbiology, vol. 20, no. 2, 2010, pages 125-129, XP055617200, ISSN: 0917-5059
- STRAPAGIEL, D. et al.: "Draft genome sequences of mycobacterium kansasii strains 1010001454, 1010001458, 1010001468, 101001493, 101001495, and 1010001469, isolated from environmental sources", Genome Announcements, vol. 4, no. 3, 2 June 2016 (2016-06-02), pages 1-2, XP055506754, ISSN: 2169-8287
- RICHTER, E. et al.: "Identification of mycobacterium kansasii by using a DNA probe (AccuProbe) and molecular techniques", Journal of Clinical Microbiology, vol. 37, no. 4, April 1999 (1999-04), pages 964-970, XP055506760, ISSN: 0095-1137

## Description

### Technical Field

The present invention relates to a primer set for amplifying a polynucleotide derived from Mycobacterium kansasii.

The present invention relates to a probe capable of specifically hybridizing to a polynucleotide derived from Mycobacterium kansasii.

The present invention relates to a kit for detecting Mycobacterium kansasii and/or a polynucleotide derived from Mycobacterium kansasii.

The present invention relates to a method for detecting Mycobacterium kansasii and/or a polynucleotide derived from Mycobacterium kansasii.

### Background Art

Mycobacterium is an anti-acidic, non-motile gram-positive bacillus. Mycobacterium is generally classified into two groups: Mycobacterium tuberculosis complex; and Nontuberculous mycobacteria (NTM).

Infection with NTM has increased worldwide in recent years. The NTM is a general term for bacteria groups in mycobacteria excluding bacteria of Mycobacterium tuberculosis complex and bacteria having specific auxotrophy. Thirty species or more NTM have been reported to cause infections (NTM diseases) in Japan.

Among causative bacteria of NTM disease, Mycobacterium avium complex (MAC) is the most common (about 70%), followed by Mycobacterium kansasii (M. kansasii) (about 10 to 20%).

M. kansasii is classified into seven types (subtypes) by hsp65RPA analysis. Type I is mostly separated from patients with respiratory disease, and Type II is separated from HIV patients. Types III to VII have been suggested to be also pathogenic.

M. kansasii is genetically closely related to M. gastri (Mycobacterium gastri) (Non Patent Literature 1). Their 16S rRNA gene sequences match with 100% (Non Patent Literature 2).

Conventionally, mycobacterial disease has been diagnosed by acid-fast staining, culturing and then performing biochemical assays for catalase production, urease activity, Tween hydrolysis, nitrate reduction, photochromic property or the like.

As determination methods for mycobacterial disease-causative bacteria other than culture methods and biochemical assays, there are reports as follows.

Non Patent Literature 3 by Huang ZH. et al. reports a method using DNA probes specific to M. kansasii.

Non Patent Literature 4 by Rogall T. et al. reports an identification method by PCR that targets 16S rRNA.

Patent Literature 1 discloses sequences unique to M. kansasii, and also discloses a detection of M. kansasii by using probe or PCR amplification that targets the sequences.

WO 2006/121134 discloses primer sets and probes for detection of M.kansasii.

### Citation List

### Patent Literature

Patent Literature 1: JP Patent Publication (Kokai) No.11-155589 A (1999)

### Non Patent Literatures

Non Patent Literature 1: Kim H. et. al, Int. J. Syst. Evol. Microbiol. 2005, 55, 1649-1656
Non Patent Literature 2: The journal of the Japanese Society for Clinical Microbiology, vol. 20, No. 2, 2010, 125-129
Non Patent Literature 3: J. Clin. Microbiol., 1991 Oct;29(10):2125-2129
Non Patent Literature 4: J. Gen. Microbiol., 1990 Sep;136(9):1915-20.
Non Patent Literature 5: Genome Announc. 2016 Jun 2; 4(3). pii: e00456-16. doi:10.1128/genomeA. 00456-16.

### Summary of Invention

### Technical Problem

As noted above, it has been known that the method for detecting and identifying M. kansasii by culturing and performing biochemical assays. However, this method has a problem that the procedure is complicated. In particular, photochromic property, which is known as an indicator for identifying M. kansasii, is not always appropriate as the identification indicator of M. kansasii, because the determination of photochromic property requires pure culture and takes time, and this property easily changes, so less reliable.

On the other hand, as described above, with focusing on nucleotide sequences of nucleic acids which M. kansasii has, the methods for detecting M. kansasii by hybridization using DNA probes or by a PCR method have been reported in Non Patent Literatures 3 and 4 and Patent Literature 1 or other documents.

However, DNA probes disclosed in Non Patent Literature 3 have problems in that, not only nucleic acids of M. kansasii, but also nucleic acids of M. gastri are cross-hybridized, while nucleic acids of genetically distant types of M. kansasii are not hybridized and cannot be detected.

The method described in Non Patent Literature 4 is a method for identification by PCR that targets 16S rRNA. As described above, since M. Kansasii and M. gastri are identical in 16S rRNA gene sequences, the method cannot detect M. kansasii with distinction from M. gastri.

Further, in the M. kansasii detection method described in Patent Literature 1, not only M. kansasii but also M. gastri is cross-hybridized, which is demonstrated in experimental results of Example 3 of Patent Literature 1.

Accordingly, an object of the present invention is to provide a new means for species-specifically detecting M. kansasii. Here, the phrase "species-specifically detecting M. kansasii" refers to, typically, a detection of M. kansasii irrespective of the type without detecting its closely related species such as M. gastri.

### Solution to Problem

The present invention discloses the following invention as a means for solving the above problem. The invention is defined by the claims. The invention concerns a primer set suitable for species-specific detection of Mycobacterium kansasii as defined in claim 1.

In preferred embodiments, the primer set is a set wherein one of the first primer and the second primer further comprises a label portion that is a label substance or a tag capable of binding to a label substance; and the other further comprises a binding portion that is a tag capable of binding to a solid phase carrier.

The invention is defined by the claims. The invention concerns a probe suitable for species-specific detection of Mycobacterium kansasii as defined in claim 3.

The claimed probe is (2p) a polynucleotide comprising a nucleotide sequence 2pb having consecutive 15 or more bases included in a nucleotide sequence 2pa identical to a nucleotide sequence represented by SEQ ID NO:38, 39 or 40 or a complementary nucleotide sequence thereof.

The probe in a preferred embodiment further comprises a label portion that is a label substance or a tag capable of binding to a label substance and/or a binding portion that is a tag capable of binding to a solid phase carrier.

The probe in a preferred embodiment further comprises a reporter fluorescent dye linked to the 5' end of the polynucleotide, and a quencher dye linked to the 3' end of the polynucleotide.

A kit for detecting Mycobacterium kansasii and/or a polynucleotide derived from Mycobacterium kansasii, the kit comprising the claimed primer set.

The kit in a preferred embodiment further comprises a solid phase carrier comprising at least in part a portion capable of binding to the binding portion.

A kit for detecting Mycobacterium kansasii and/or a polynucleotide derived from Mycobacterium kansasii, in a preferred embodiment further comprises the claimed probe.

A method for detecting Mycobacterium kansasii and/or a polynucleotide derived from Mycobacterium kansasii in a sample, the method comprising
detecting a polynucleotide in the sample, the polynucleotide comprising a first nucleotide sequence represented by SEQ ID NO:1, 9, 11, 13, 15, 2, 10, 12, 14 or 16; or a second nucleotide sequence complementary to the first nucleotide sequence; or a third nucleotide sequence comprising the first nucleotide sequence or second nucleotide sequence in which any T is replaced with U. comprising
performing a nucleic acid amplification reaction on a polynucleotide in a sample as a template by using the claimed primer set, and
detecting a polynucleotide fragment amplified by the nucleic acid amplification reaction.

The above method which comprises the following step incubating a polynucleotide in a sample or a polynucleotide fragment amplified by performing a nucleic acid amplification reaction on a polynucleotide in a sample as a template with the claimed probe under a condition allowing hybridization, and detecting a hybridization of the polynucleotide or the polynucleotide fragment with the probe.

### Advantageous Effects of Invention

According to the M. kansasii detection means provided by the present invention, it is possible to reduce the possibilities of false negatives due to failure to detect M. kansasii and false positives due to misidentification of M. gastri, and possible to enhance the accuracy of identification of M. kansasii.

### Brief Description of Drawings

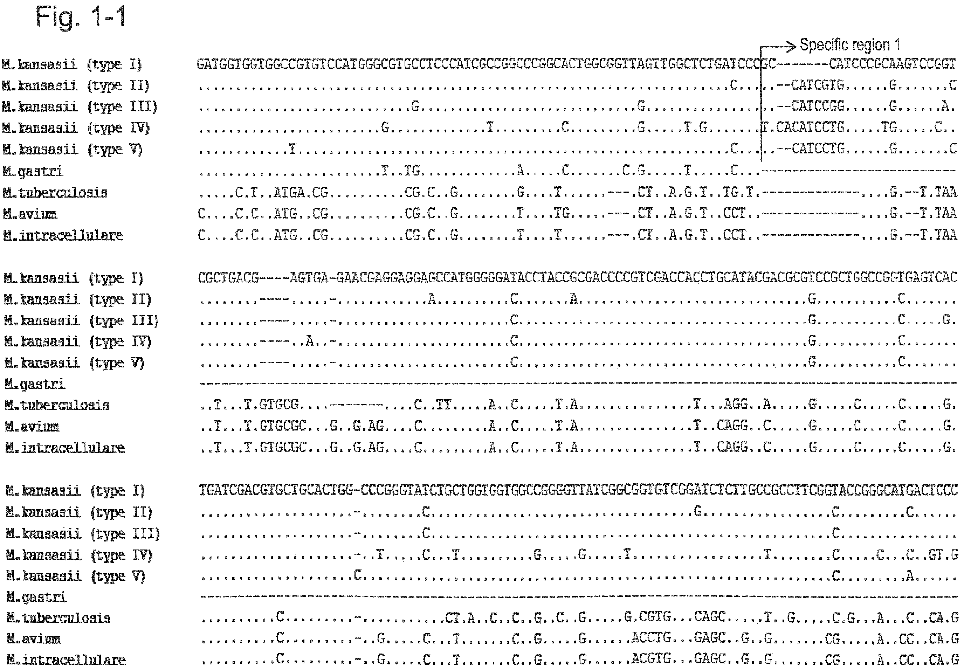

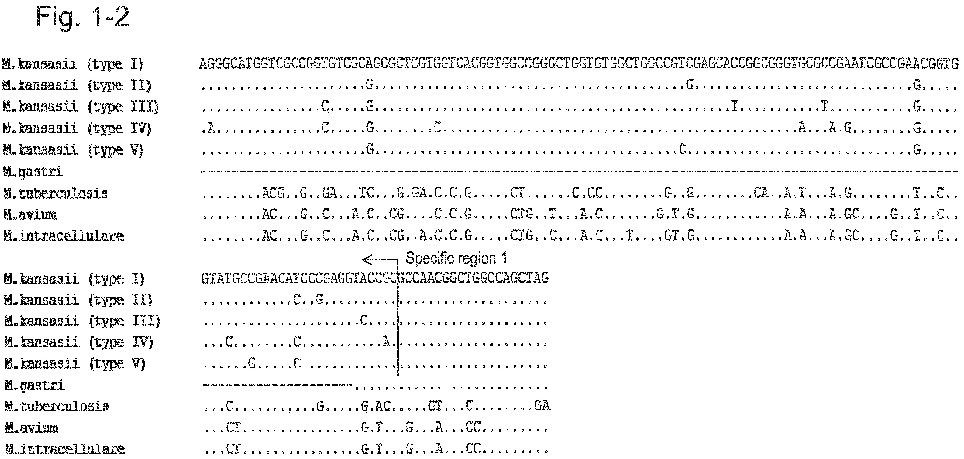

[Figure 1-1] Figure 1-1 shows a comparison result of genomic DNAs of different types of M. kansasii and other mycobacteria in the proximity of specific region 1.
[Figure 1-2] Figure 1-2 is a continuation of Figure 1-1.
[Figure 2-1] Figure 2-1 shows a comparison result of genomic DNAs of different types of M. kansasii and other mycobacteria in the proximity of specific region 2.
[Figure 2-2] Figure 2-2 is a continuation of Figure 2-1.

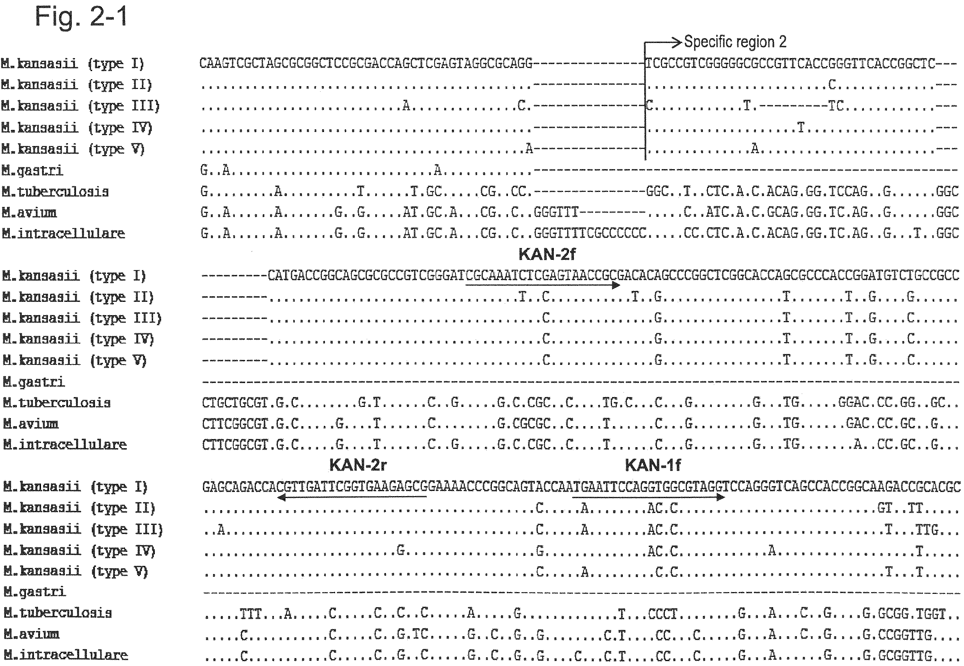

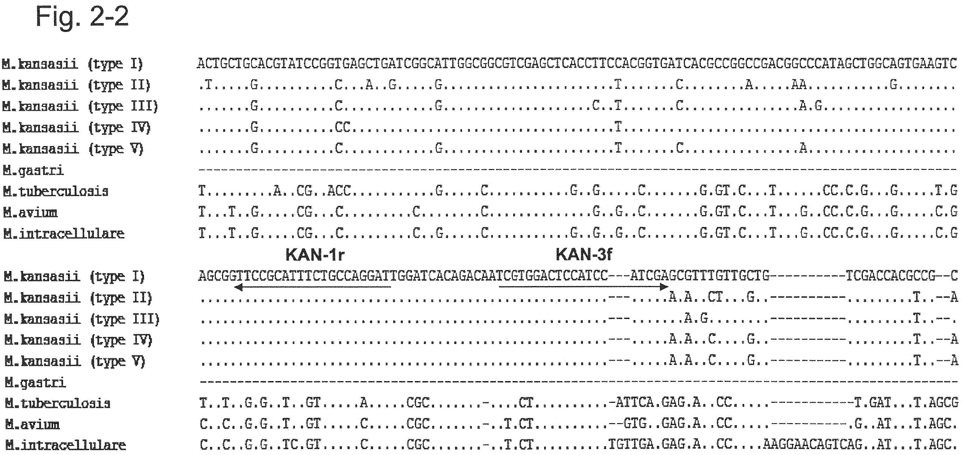

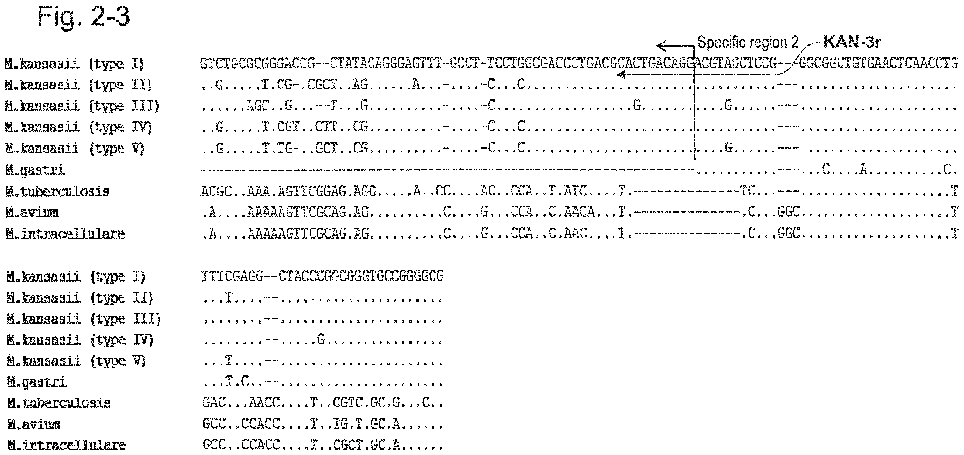

[Figure 2-3] Figure 2-3 is a continuation of Figure 2-2.

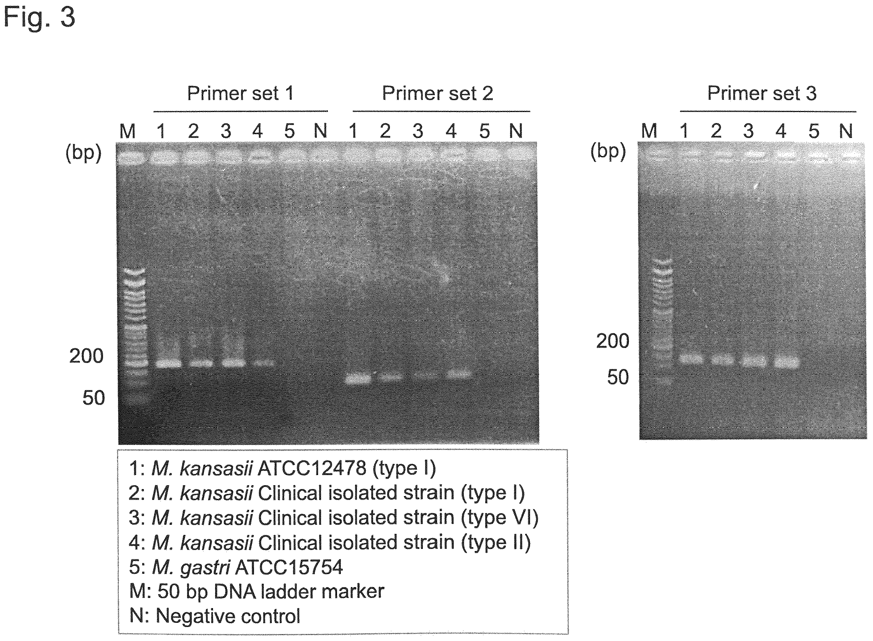

[Figure 3] Figure 3 is a photograph showing a result of analyzing the PCR reaction solution obtained in Example 1 by agarose electrophoresis.

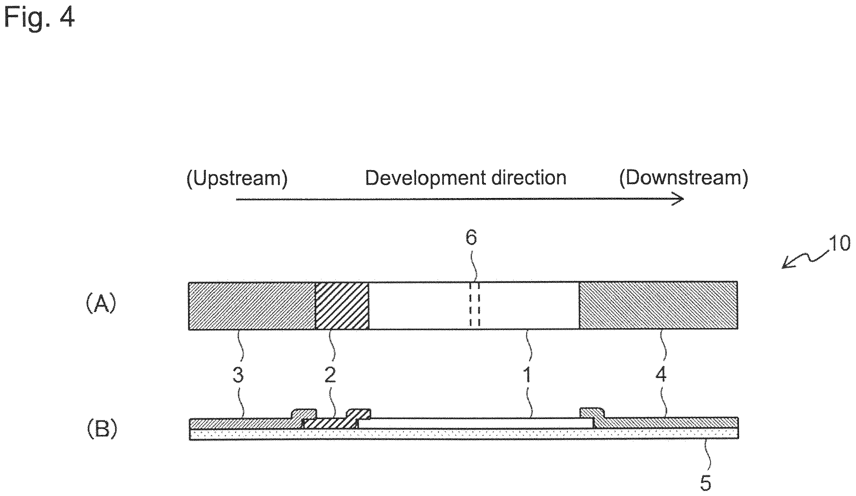

[Figure 4] Figure 4 shows a schematic view of a lateral flow type nucleic acid detection device 10. 1: solid phase carrier, 2: conjugate pad (label substance holding portion), 3: sample pad (reaction mixture receiving portion), 4: absorbent pad, 5: substrate, 6: portion having tag capture means in the solid phase carrier 1.

### Description of Embodiments

Hereinafter, the present invention will be described in detail.

### <1. Genomic DNA of Mycobacterium kansasii>

Draft genome sequences of type (or subtype) I, II, III, IV and V of M. kansasii have been disclosed in Non Patent Literature 5 published in 2016.

The draft genome sequences disclosed in Non Patent Literature 5 and registered in GenBank are as follows.

**[Table 1]**

| Strain | Type | GenBank accession number (version) |
|---|---|---|
| 1010001495 | I | LWCL00000000 (LWCL00000000.1) |
| 1010001469 | II | LWCM00000000 (LWCM00000000.1) |
| 1010001468 | III | LWCJ00000000 (LWCJ00000000.1) |
| 1010001458 | IV | LWCI00000000 (LWCI00000000.1) |
| 1010001454 | V | LWCH00000000 (LWCH00000000.1) |
| 1010001493 | V | LWCK00000000 (LWCK00000000.1) |

Using this published information, the present inventors searched a region in the genomic DNA which is highly conserved in each type of M. kansasii and differ from the other mycobacteria. As a result, the present inventors have found the regions upstream and downstream of SigF gene, the regions being highly conserved in the genomic DNA of M. kansasii and having no high homology sequences to the genomic DNA of M. gastri. Hereinafter, the region upstream of SigF gene refers to "specific region 1" and the region downstream of SigF gene refers to "specific region 2".

As to the genomic DNA of M. kansasii Type I (GenBank Accession No. LWCL00000000), the nucleotide sequence of the specific region 1 is represented by SEQ ID NO:1, and the nucleotide sequence of the specific region 2 is represented by SEQ ID NO:2. Of the genomic DNA of M. kansasii Type I, the partial nucleotide sequence of a portion including the specific region 1 and the specific region 2 is represented by SEQ ID NO:17. In SEQ ID NO:17, the region from g at position 1520 to g at position 1852 corresponds to the specific region 1, and the region from t at position 3532 to t at position 4006 corresponds to the specific region 2.

As to the genomic DNA of M. kansasii Type II (GenBank Accession No. LWCM00000000), the nucleotide sequence of the specific region 1 is represented by SEQ ID NO:9, and the nucleotide sequence of the specific region 2 is represented by SEQ ID NO:10. Of the genomic DNA of M. kansasii Type II, the partial nucleotide sequence of a portion including the specific region 1 and the specific region 2 is represented by SEQ ID NO:18. In SEQ ID NO:18, the region from g at position 1541 to g at position 1878 corresponds to the specific region 1, and the region from t at position 3565 to g at position 4040 corresponds to the specific region 2.

As to the genomic DNA of M. kansasii Type III (GenBank Accession No. LWCJ00000000), the nucleotide sequence of specific region 1 is represented by SEQ ID NO:11, and the nucleotide sequence of specific region 2 is represented by SEQ ID NO:12. Of the genomic DNA of M. kansasii Type III, the partial nucleotide sequence of a portion including the specific region 1 and the specific region 2 is represented by SEQ ID NO:19. In SEQ ID NO:19, the region from g at position 1545 to g at position 1882 corresponds to the specific region 1, and the region from c at position 3570 to g at position 4035 corresponds to the specific region 2.

As to the genomic DNA of M. kansasii Type IV (GenBank Accession No. LWCI00000000), the nucleotide sequence of specific region 1 is represented by SEQ ID NO:13, and the nucleotide sequence of specific region 2 is represented by SEQ ID NO:14. Of the genomic DNA of M. kansasii Type IV, the partial nucleotide sequence of a portion including the specific region 1 and the specific region 2 is represented by SEQ ID NO:20. In SEQ ID NO:20, the region from t at position 1552 to g at position 1891 corresponds to the specific region 1, and the region from t at position 3571 to t at position 4047 corresponds to the specific region 2.

As to the genomic DNA of M. kansasii Type V (GenBank Accession No. LWCH00000000), the nucleotide sequence of specific region 1 is represented by SEQ ID NO:15, and the nucleotide sequence of specific region 2 is represented by SEQ ID NO:16. Of the genomic DNA of M. kansasii Type V, the partial nucleotide sequence of a portion including the specific region 1 and the specific region 2 is represented by SEQ ID NO:21. In SEQ ID NO:21, the region from g at position 1551 to g at position 1889 corresponds to the specific region 1, and the region from t at position 3578 to g at position 4053 corresponds to the specific region 2.

In addition, the comparison results in the proximity of specific region 1 and the comparison results in the proximity of specific region 2 of genomic DNAs between different types of M. kansasii and M. gastri, Mycobacterium (M.) tuberculosis (Mycobacterium tuberculosis), M. avium, M. intracellulare are shown in Figure 1 (consisting of Figures 1-1 and 1-2) and Figure 2 (consisting of Figs 2-1, 2-2 and 2-3), respectively.

The present inventors have found that using the specific region 1 or the specific region 2 in a polynucleotide derived from genomic DNA as an indicator, it is possible to detect M. kansasii irrespective of the type without detecting its closely related species such as M. gastri, and have completed the present invention.

### <2. Terms>

In the present invention, the term "polynucleotide" refers to deoxyribonucleic acid (DNA) or ribonucleic acid (RNA). In RNA, thymine (T) can be read as uracil (U). A DNA containing U which is synthesized by substituting T at any position with U, and an RNA containing T which is synthesized by substituting U at any position with T also may be used as a polynucleotide. A modified nucleotide such as inosine (I) may be included in part in a polynucleotide.

The polynucleotide may be present as a single strand or a double strand. When the polynucleotide is present as a double strand, it is enough that at least one of the strand is a polynucleotide with a predetermined characteristic as defined below.

The method of preparing the polynucleotide is not particularly limited, and the polynucleotide may be prepared by using a polynucleotide synthesizer, or may be prepared by using a custom synthesis services.

As used herein, the phrase "a nucleotide sequence Y homologous to a nucleotide sequence X" or "a nucleotide sequence X and a nucleotide sequence Y are homologous to each other" includes the case the nucleotide sequences X and Y are different partially from each other as long as a polynucleotide consisting of the complementary sequence of the nucleotide sequence X and a polynucleotide consisting of the nucleotide sequence Y form a combination capable of hybridizing to form a hydrogen bonding enough to form a stable duplex in an annealing condition of nucleic acid amplification reaction. For example, there may be some mismatches between the polynucleotide consisting of the complementary sequence of the nucleotide sequence X and the polynucleotide consisting of the nucleotide sequence Y, such as 1 mismatch in 10 nucleotides, 1 mismatch in 20 nucleotides, 1 mismatch in 30 nucleotides, or other probabilities. Typically, the nucleotide sequence Y "homologous" to nucleotide sequence X means that the nucleotide sequences X and Y meet any of the following relations (A) to (D).
(A) The nucleotide sequence Y is a nucleotide sequence in which one or several bases are deleted, substituted, added and/or inserted in the nucleotide sequence X.
(B) The nucleotide sequence Y is a nucleotide sequence having an identity of 70% or more to the nucleotide sequence X.
(C) The polynucleotide consisting of the nucleotide sequence Y can be hybridized with the polynucleotide consisting of a nucleotide sequence complementary to the nucleotide sequence X under a stringent condition.
(D) Thymine (T) at any position in one of the nucleotide sequences X and Y is substituted by uracil (T) in the other.

In the (A), the terms "one or several" represents preferably 1 to 5, more preferably 1 to 4, more preferably 1 to 3, particularly preferably 1 or 2, most preferably 1.

In the (B), the value of identity means a value calculated in a default setting using a software for calculating identity between multiple nucleotide sequences (for example, FASTA, DNASIS, and BLAST). The identity value of nucleotide sequences is obtained by determining the number of bases matched upon aligning a pair of nucleotide sequences so as to maximize the matching degree, and calculating a ratio by dividing the number of matched bases by the total number of the bases in the nucleotide sequence which has been compared. If there is a gap, the total number of bases described above is a number of bases obtained by counting one gap as one base. See, for example, Altschul et al, Nuc. Acids. Res. 25, 3389-3402, 1977 and Altschul et al, J. Mol. Biol. 215, 403-410,1990, for details of the determination method of identity.

In the (B), the identity is more preferably an identity of 80% or more, more preferably of 90% or more, more preferably of 95% or more, more preferably of 96% or more, more preferably of 97% or more, more preferably of 98% or more, more preferably of 99% or more.

In the (C), "stringent condition" means a condition in which a so-called specific hybrid is formed and non-specific hybrid is not formed, and it can be appropriately set, for example, with reference to Green and Sambrook, Molecular Cloning, 4th Ed (2012), Cold Spring Harbor Laboratory Press. Specifically, a stringent condition can be set by the temperature and the salt concentration in the solution upon southern hybridization, and the temperature and the salt concentration in the solution during the washing step of southern hybridization. More specifically, examples of the stringent condition include, for example, in a hybridization step, the sodium concentration of 25 to 500 mM, preferably 25 to 300 mM, and a temperature of 40 to 68°C, preferably 40 to 65°C. More specifically, hybridization can be performed in 1 to 7 × SSC, 0.02 to 3% SDS, and the temperature of 40°C to 60°C. In addition, a washing step may be performed after hybridization. The washing step can be performed, for example, in 0.1 to 2 × SSC, 0.1 to 0.3% SDS, and the temperature of 50 to 65°C.

### <3. Primer set>

The present invention relates to a primer set as defined in the claims comprising:
a first primer comprising a polynucleotide comprising, at the 3' end, a nucleotide sequence A' identical or homologous to a nucleotide sequence A that is a partial nucleotide sequence at the 5' end of a target region in the genomic DNA of M. kansasii, the target region comprising all or a part of a first nucleotide sequence represented by SEQ ID NO:1, 9, 11, 13, 15, 2, 10, 12, 14 or 16, or all or a part of a second nucleotide sequence complementary to the first nucleotide sequence; and
a second primer comprising a polynucleotide comprising, at the 3' end, a nucleotide sequence B' identical or homologous to a complementary nucleotide sequence to a nucleotide sequence B that is a partial nucleotide sequence at the 3' end of the target region.

The genomic DNA of M. kansasii is as described above.

The "first nucleotide sequence" corresponds to a nucleotide sequence of specific region 1 or specific region 2 on the genomic DNA. The "second nucleotide sequence" corresponds to a complementary nucleotide sequence to the nucleotide sequence of specific region 1 or specific region 2.

The primer set of the present invention is designed so as to allow an amplification by a nucleic acid amplification reaction of a region comprising all or a part of the first nucleotide sequence or the second nucleotide sequence in the genomic DNA of M. kansasii (the region is referred to as "target region").

The target region may be a region consisting of only all or a part of the first nucleotide sequence or the second nucleotide sequence in the genomic DNA, or may be a consecutive region consisting of all or a part of the first nucleotide sequence or the second nucleotide sequence and one or more other parts adjacent thereto in the genomic DNA. The other part(s) may be positioned in the upstream (the 5' end) side of the first nucleotide sequence or the second nucleotide sequence, or may be positioned in the downstream (the 3' end) side thereof, or may include both of the upstream and downstream positioned parts in the genomic DNA. For example, when the upstream side end of a target region is positioned in a part adjacent to the upstream side of the first nucleotide sequence or the second nucleotide sequence and the downstream side end of the target region is positioned in a part adjacent to the downstream side of the first nucleotide sequence or the second nucleotide sequence, the target region is a consecutive region including all of the first nucleotide sequence or the second nucleotide sequence, and the part adjacent to the upstream side thereof and the part adjacent to the downstream side thereof in the genomic DNA.

For example, the target region amplified by primer set 1 described below is a consecutive region consisting of only a part of the specific region 2 in the genomic DNA, which corresponds to a region of 176 bases of position 3719 to position 3885 of a nucleotide sequence represented by SEQ ID NO:17 (and a complementary strand of the region) in the genomic DNA of M. kansasii Type I. The target region amplified by primer set 2 described below is a consecutive region consisting of only a part of the specific region 2 in the genomic DNA, which corresponds to a region of 95 bases of position 3596 to position 3690 of a nucleotide sequence represented by SEQ ID NO:17 (and a complementary strand of the region) in the genomic DNA of M. kansasii Type I. The target region amplified by primer set 3 described below is a consecutive region consisting of a part of the specific region 2 and another part adjacent to the downstream side of the part in the genomic DNA, which corresponds to a region of 117 bases of position 3900 to position 4016 of a nucleotide sequence represented by SEQ ID NO:17 (and a complementary strand of the region) in the genomic DNA of M. kansasii Type I.

The target region may be a region in the genomic DNA including both of all or a part of the specific region 1 and all or a part of the specific region 2.

The length of target region is not particularly limited. To enhance detection accuracy, it is preferred that the target region comprises a consecutive part of preferably 20 or more bases, more preferably 30 or more bases, more preferably 40 or more bases, more preferably 50 or more bases, more preferably 60 or more bases of the first nucleotide sequence or the second nucleotide sequence. The entire length of the target region can be, for example, 40 or more bases, preferably 50 or more bases, more preferably 60 or more bases, and more preferably 70 or more bases, and the upper limit of the length is not particularly limited, but usually, 1000 or less bases, or 500 or less bases.

The first primer comprised in the primer set of the present invention comprises a polynucleotide comprising, at the 3' end, a nucleotide sequence A' identical or homologous to a nucleotide sequence A that is a partial nucleotide sequence at the 5' end of a target region in the genomic DNA of M. kansasii.

The nucleotide sequence A is a "partial nucleotide sequence of the 5' end" of a target sequence, specifically, is a partial nucleotide sequence in a consecutive part from the base at the 5' end toward downstream side of the target sequence. The length of nucleotide sequence A may be, for example, 8 or more bases, preferably 10 or more bases, more preferably 12 or more bases, more preferably 15 or more bases, and typically 40 or less bases, 30 or less bases, or 25 or less bases.

The polynucleotide in the first primer can be designed so as to comprise at the 3' end a nucleotide sequence A' identical or homologous to the nucleotide sequence A. The "homologous" here is as described above. The polynucleotide in the first primer comprises the nucleotide sequence A' at the 3' end, and it may further comprise another nucleotide sequence at the 5' end side of the nucleotide sequence A'. The entire length of polynucleotide in the first primer is not particularly limited, but may be, for example, 8 or more bases, preferably 10 or more bases, more preferably 12 or more bases, more preferably 15 or more bases, and typically 40 or less bases, 30 or less bases, or 25 or less bases.

The second primer comprised in the primer set of the present invention comprises a polynucleotide comprising, at the 3' end, a nucleotide sequence B' identical or homologous to a complementary nucleotide sequence to a nucleotide sequence B that is a partial nucleotide sequence at the 3' end of the target region in the genomic DNA of M. kansasii.

The nucleotide sequence B is a "partial nucleotide sequence at the 3' end" of a target sequence, specifically a partial nucleotide sequence in a consecutive part from the base at the 3' end toward the upstream side of the target sequence. The length of nucleotide sequence B may be, for example, 8 or more bases, preferably 10 or more bases, more preferably 12 or more bases, more preferably 15 or more bases, and typically 40 or less bases, 30 or less bases, or 25 or less bases.

It is preferred that the nucleotide sequence B is a partial nucleotide sequence, among the genomic DNA of M. kansasii, in the specific regions 1 and 2

The nucleotide sequence B is set so as to position the 5' end base of the nucleotide sequence B in more downstream than the 5' end base of the nucleotide sequence A in the target region. Preferably, the nucleotide sequence B is set so as to position the 5' end base of the nucleotide sequence B in more downstream than the 3' end base of the nucleotide sequence A in the target region.

The combination of the first primer and the second primer is not particularly limited, and they can be combined to allow the target region of the genomic DNA to be amplified as a polynucleotide fragment.

In the present invention, specific preferred examples of a primer set for amplifying a target region comprising a part of the specific region 2, that is a first nucleotide sequence represented by SEQ ID NO:2, 10, 12, 14 or 16 or a second nucleotide sequence complementary to the first nucleotide sequence in the genomic DNA of M. kansasii, as a polynucleotide fragment, include the primer set, wherein
the first primer comprises
(2f) a polynucleotide having 40 or less bases comprising, at the 3' end, a nucleotide sequence 2fb having consecutive 8 or more bases included in a nucleotide sequence 2fa identical or homologous to a nucleotide sequence represented by SEQ ID NO:3, 5 or 7, and the second primer comprises
(2r) a polynucleotide having 40 or less bases comprising, at the 3' end, a nucleotide sequence 2rb having consecutive 8 or more bases included in a nucleotide sequence 2ra identical or homologous to a nucleotide sequence represented by SEQ ID NO:4, 6 or 8.

SEQ ID NO:3 corresponds to position 3710 to position 3729 of SEQ ID NO: 17 (shown as KAN-1f in Figure 2-1).

SEQ ID NO:5 corresponds to position 3596 to position 3615 of SEQ ID NO: 17 (shown as KAN-2f in Figure 2-1).

SEQ ID NO:7 corresponds to position 3900 to position 3919 of SEQ ID NO:17 (shown as KAN-3f in Figure 2-2).

SEQ ID NO:4 corresponds to a complementary nucleotide sequence to position 3866 to position 3885 of SEQ ID NO: 17 (shown as KAN-1r in Figure 2-2).

SEQ ID NO:6 corresponds to a complementary nucleotide sequence to position 3671 to position 3690 of SEQ ID NO:17 (shown as KAN-2r in Figure 2-1).

SEQ ID NO:8 corresponds to a complementary nucleotide sequence to position 3997 to position 4016 of SEQ ID NO:17 (shown as KAN-3r in Figure 2-3).

In the (2f), the nucleotide sequence 2fa is identical to the nucleotide sequence represented by SEQ ID NO:3, 5 or 7, or is homologous to them, in other words, satisfies the relation of the (A), (B), (C) or (D) (the nucleotide sequence represented by SEQ ID NO:3, 5 or 7 corresponds to the nucleotide sequence X, and the nucleotide sequence 2fa corresponds to the nucleotide sequence Y).

In the case that the nucleotide sequence 2fa satisfies the relation of the (A) with the nucleotide sequence represented by SEQ ID NO:3, 5 or 7, it is more preferred that the nucleotide sequence 2fa is a sequence in which one or several bases in total are deleted from either or both of the 5' end and the 3' end in the nucleotide sequence represented by SEQ ID NO:3, 5 or 7, and it is particularly preferred that the nucleotide sequence 2fa is a sequence in which one or several bases in total are deleted from the 5' end only in the nucleotide sequence represented by SEQ ID NO:3, 5 or 7. In the case that the nucleotide sequence 2fa satisfies the relation of the (A) with the nucleotide sequence represented by SEQ ID NO:3, 5 or 7, it is more preferred that the nucleotide sequence 2fa is a sequence in which one or several bases in total are added to either or both of the 5' end and the 3' end in the nucleotide sequence represented by SEQ ID NO:3, 5 or 7, and it is particularly preferred that the nucleotide sequence 2fa is a partial nucleotide sequence of a the nucleotide sequence represented by SEQ ID NO:17, wherein the partial nucleotide sequence consists of the nucleotide sequence represented by SEQ ID NO:3, 5 or 7 and one or several bases in total added to either or both of the 5' end and the 3' end of the nucleotide sequence represented by SEQ ID NO:3, 5 or 7.

Other preferred examples of the cases that the nucleotide sequence 2fa satisfies the relation of the (A) with the nucleotide sequence represented by SEQ ID NO:3, 5 or 7 include, for example, the following embodiment. As described above, each of the nucleotide sequences represented by SEQ ID NO:3, 5 and 7 is a partial nucleotide sequence in the nucleotide sequence represented by SEQ ID NO:17 in the DNA of M. kansasii Type I. On the other hand, the DNA of other types of M. kansasii comprises the nucleotide sequence represented by SEQ ID NO:18, 19, 20 or 21, corresponding to the SEQ ID NO:17, and in the nucleotide sequence, a partial nucleotide sequence corresponding to the nucleotide sequence represented by SEQ ID NO:3, 5 or 7 is present. Then, the partial nucleotide sequences corresponding to the nucleotide sequence represented by SEQ ID NOS: 3, 5 and 7 in the nucleotide sequence represented by SEQ ID NO: 18, 19, 20 or 21, are set as partial nucleotide sequences 3', 5' and 7', respectively, upon aligning the nucleotide sequence represented by SEQ ID NO:18, 19, 20 or 21 with the nucleotide sequence represented by SEQ ID NO:17 so as to maximize the matching degree of the nucleotide sequence in the specific region 2. When the partial nucleotide sequence 3', 5' or 7' is a nucleotide sequence containing one or more modifications in which one or several bases are deleted, substituted, added and/or inserted in the nucleotide sequence represented by SEQ ID NO:3, 5 or 7, respectively, a nucleotide sequence in which at least one of the modifications (deletion, substitution, addition and/or insertion) are introduced into the nucleotide sequence represented by SEQ ID NO:3, 5 or 7 can be set as nucleotide sequence 2fa, and more preferably, the partial nucleotide sequence 3', 5' or 7' can be set as nucleotide sequence 2fa.

In the (2f), it is particularly preferred that nucleotide sequence 2fa is identical to a nucleotide sequence represented by SEQ ID NO:3, 5 or 7.

In the (2f), the nucleotide sequence 2fb is a nucleotide sequence contained in the nucleotide sequence 2fa having consecutive 8 or more bases, preferably 10 or more bases, more preferably 12 or more bases, more preferably 15 or more bases, and the nucleotide sequence 2fb may be identical to the nucleotide sequence 2fa or may be a partial nucleotide sequence of the nucleotide sequence 2fa. The nucleotide sequence 2fb is preferably a consecutive nucleotide sequence comprising a base of the 3' end contained in the nucleotide sequence 2fa.

In the (2f), it is particularly preferred that the nucleotide sequence 2fb is identical to the nucleotide sequence 2fa.

The polynucleotide of (2f) comprises the nucleotide sequence 2fb at the 3' end, and it may further comprise another nucleotide sequence at the 5' end side of the nucleotide sequence 2fb. The polynucleotide of (2f) is a polynucleotide having 40 or less bases, preferably 35 or less bases, more preferably 30 or less bases, more preferably 25 or less bases, particularly preferably 22 or less bases.

It is particularly preferred that the polynucleotide of (2f) consists of the nucleotide sequence 2fb.

In the (2r), the nucleotide sequence 2ra is identical to the nucleotide sequence represented by SEQ ID NO:4, 6 or 8, or is homologous to them, in other words, satisfies the relation of the (A), (B), (C) or (D) (the nucleotide sequence represented by SEQ ID NO:4, 6 or 8 corresponds to the nucleotide sequence X, and the nucleotide sequence 2ra corresponds to the nucleotide sequence Y).

In the case that the nucleotide sequence 2ra satisfies the relation of the (A) with the nucleotide sequence represented by SEQ ID NO:4, 6 or 8, it is more preferred that the nucleotide sequence 2ra is a sequence in which one or several bases in total are deleted from either or both of the 5' end and the 3' end in the nucleotide sequence represented by SEQ ID NO:4, 6 or 8, and it is particularly preferred that the nucleotide sequence 2ra is a sequence in which one or several bases in total are deleted from the 5' end only in the nucleotide sequence represented by SEQ ID NO:4, 6 or 8. In the case that the nucleotide sequence 2ra satisfies the relation of the (A) with the nucleotide sequence represented by SEQ ID NO:4, 6 or 8, it is more preferred that the nucleotide sequence 2ra is a sequence in which one or several bases in total are added to either or both of the 5' end and the 3' end in the nucleotide sequence represented by SEQ ID NO:4, 6 or 8, and it is particularly preferred that the nucleotide sequence 2ra is a partial nucleotide sequence of a complementary nucleotide sequence to the nucleotide sequence represented by SEQ ID NO: 17, wherein the partial nucleotide sequence consists of the nucleotide sequence represented by SEQ ID NO:4, 6 or 8 and one or several bases in total added to either or both of the 5' end and the 3' end of the nucleotide sequence represented by SEQ ID NO:4, 6 or 8.

Other preferred examples of the cases that the nucleotide sequence 2ra satisfies the relation of the (A) with the nucleotide sequence represented by SEQ ID NO:4, 6 or 8 include, for example, the following embodiment. The partial nucleotide sequences corresponding to the nucleotide sequence represented by SEQ ID NOS:4, 6 and 8 in a complementary nucleotide sequence to the nucleotide sequence represented by SEQ ID NO: 18, 19, 20 or 21, are set as partial nucleotide sequences 4', 6' and 8', respectively, upon aligning the complementary nucleotide sequence to the nucleotide sequence represented by SEQ ID NO: 18, 19, 20 or 21 with a complementary nucleotide sequence to the nucleotide sequence represented by SEQ ID NO:17 so as to maximize the matching degree of the complementary nucleotide sequence in the specific region 2. When the partial nucleotide sequence 4', 6' or 8' is a nucleotide sequence containing one or more modifications in which one or several bases are deleted, substituted, added and/or inserted in the nucleotide sequence represented by SEQ ID NO:4, 6 or 8, respectively, a nucleotide sequence in which at least one of the modifications (deletion, substitution, addition and/or insertion) are introduced into the nucleotide sequence represented by SEQ ID NO:4, 6 or 8 can be set as nucleotide sequence 2ra, and more preferably, the partial nucleotide sequence 4', 6' or 8' can be set as nucleotide sequence 2ra.

In the (2r), it is particularly preferred that the nucleotide sequence 2ra is identical to a nucleotide sequence represented by SEQ ID NO:4, 6 or 8.

In the (2r), the nucleotide sequence 2rb is a nucleotide sequence contained in the nucleotide sequence 2ra having consecutive 8 or more bases, preferably 10 or more bases, more preferably 12 or more bases, more preferably 15 or more bases, and the nucleotide sequence 2rb may be identical to the nucleotide sequence 2ra or may be a partial nucleotide sequence of the nucleotide sequence 2ra. The nucleotide sequence 2rb is preferably a consecutive nucleotide sequence comprising a base of the 3' end contained in the nucleotide sequence 2ra.

In the (2r), it is particularly preferred that nucleotide sequence 2rb is identical to nucleotide sequence 2ra.

The polynucleotide of (2r) comprises the nucleotide sequence 2rb at the 3' end, and it may further comprise another nucleotide sequence at the 5' end side of the nucleotide sequence 2rb. The polynucleotide of (2r) is a polynucleotide having 40 or less bases, preferably 35 or less bases, more preferably 30 or less bases, more preferably 25 or less bases, particularly preferably 22 or less bases.

It is particularly preferred that the polynucleotide of (2r) consists of nucleotide sequence 2rb.

The combination of the first primer comprising the polynucleotide of (2f) and the second primer comprising the polynucleotide of (2r) is not particularly limited, and they can be combined to allow the target region of the genomic DNA to be amplified as a polynucleotide fragment. Specific examples of the combination include the following.

When the "nucleotide sequence represented by SEQ ID NO:3, 5 or 7" in the (2f) is the "nucleotide sequence represented by SEQ ID NO:3", the "nucleotide sequence represented by SEQ ID NO:4, 6 or 8" in the (2r) is "nucleotide sequence represented by SEQ ID NO:4 or 8", and preferably the "nucleotide sequence represented by SEQ ID NO:4". In particular, it is preferably a combination of the polynucleotide of (2f) which is a polynucleotide (KAN-1f) consisting of a nucleotide sequence represented by SEQ ID NO:3 and the polynucleotide of (2r) which is a polynucleotide (KAN-1r) consisting of a nucleotide sequence represented by SEQ ID NO:4, the combination being "primer set 1".

When the "nucleotide sequence represented by SEQ ID NO:3, 5 or 7" in the (2f) is the "nucleotide sequence represented by SEQ ID NO:5", the "nucleotide sequence represented by SEQ ID NO:4, 6 or 8" in the (2r) may be any of them, but preferably the "nucleotide sequence represented by SEQ ID NO:6". In particular, it is preferably a combination of the polynucleotide of (2f) which is a polynucleotide (KAN-2f) consisting of a nucleotide sequence represented by SEQ ID NO:5 and the polynucleotide of (2r) which is a polynucleotide (KAN-2r) consisting of a nucleotide sequence represented by SEQ ID NO:6, the combination being "primer set 2".

When the "nucleotide sequence represented by SEQ ID NO:3, 5 or 7" in the (2f) is the "nucleotide sequence represented by SEQ ID NO:7", the "nucleotide sequence represented by SEQ ID NO:4, 6 or 8" in the (2r) is the "nucleotide sequence represented by SEQ ID NO:8". In particular, it is preferably a combination of the polynucleotide of (2f) which is a polynucleotide (KAN-3f) consisting of a nucleotide sequence represented by SEQ ID NO:7 and the polynucleotide of (2r) which is a polynucleotide (KAN-3r) consisting of a nucleotide sequence represented by SEQ ID NO:8, the combination being "primer set 3".

In the present invention, preferred specific examples of a primer set for amplifying a target region comprising a part of the specific region 1, that is a first nucleotide sequence represented by SEQ ID NO:1, 9, 11, 13, or 15 or a second nucleotide sequence complementary to the first nucleotide sequence in the genomic DNA of M. kansasii, as a polynucleotide fragment, include the primer set, wherein
the first primer comprises
   (1f) a polynucleotide having 40 or less bases comprising, at the 3' end, a nucleotide sequence lfb having consecutive 8 or more bases included in a nucleotide sequence 1fa identical to a nucleotide sequence represented by SEQ ID NO:22, 24, 26, 28, 30, 32, 34 or 36, and
the second primer comprises
   (1r) a polynucleotide having 40 or less bases comprising, at the 3' end, a nucleotide sequence 1rb having consecutive 8 or more bases included in a nucleotide sequence 1ra identical to a nucleotide sequence represented by SEQ ID NO:23, 25, 27, 29, 31, 33, 35 or 37.

SEQ ID NO:22 corresponds to position 1544 to position 1563 of SEQ ID NO:17.

SEQ ID NO:24 corresponds to position 1549 to position 1568 of SEQ ID NO:17.

SEQ ID NO:26 corresponds to position 1541 to position 1561 of SEQ ID NO:17.

SEQ ID NO:28 corresponds to position 1682 to position 1701 of SEQ ID NO:17.

SEQ ID NO:30 corresponds to position 1538 to position 1556 of SEQ ID NO:17.

SEQ ID NO:32 corresponds to position 1538 to position 1557 of SEQ ID NO:17.

SEQ ID NO:34 corresponds to position 1683 to position 1702 of SEQ ID NO:17.

SEQ ID NO:36 corresponds to position 1724 to position 1742 of SEQ ID NO:17.

SEQ ID NO:23 corresponds to a complementary nucleotide sequence to position 1826 to position 1845 of SEQ ID NO:17.

SEQ ID NO:25 corresponds to a complementary nucleotide sequence to position 1825 to position 1844 of SEQ ID NO:17.

SEQ ID NO:27 corresponds to a complementary nucleotide sequence to position 1682 to position 1700 of SEQ ID NO:17.

SEQ ID NO:29 corresponds to a complementary nucleotide sequence to position 1824 to position 1842 of SEQ ID NO:17.

SEQ ID NO:31 corresponds to a complementary nucleotide sequence to position 1682 to position 1701 of SEQ ID NO:17.

SEQ ID NO:33 corresponds to a complementary nucleotide sequence to position 1589 to position 1607 of SEQ ID NO:17.

SEQ ID NO:35 corresponds to a complementary nucleotide sequence to position 1823 to position 1842 of SEQ ID NO:17.

SEQ ID NO:37 corresponds to a complementary nucleotide sequence to position 1824 to position 1844 of SEQ ID NO: 17.

In the (1f), the nucleotide sequence 1fa is identical to the nucleotide sequence represented by SEQ ID NO:22, 24, 26, 28, 30, 32, 34 or 36, in other words, satisfies the relation of the (A), (B), (C) or (D) (the nucleotide sequence represented by SEQ ID NO:22, 24, 26, 28, 30, 32, 34 or 36 corresponds to the nucleotide sequence X, and the nucleotide sequence 1fa corresponds to the nucleotide sequence Y).

In the case that the nucleotide sequence 1fa satisfies the relation of the (A) with the nucleotide sequence represented by SEQ ID NO:22, 24, 26, 28, 30, 32, 34 or 36, it is more preferred that the nucleotide sequence 1fa is a sequence in which one or several bases in total are deleted from either or both of the 5' end and the 3' end in the nucleotide sequence represented by SEQ ID NO:22, 24, 26, 28, 30, 32, 34 or 36, and it is particularly preferred that the nucleotide sequence 1fa is a sequence in which one or several bases in total are deleted from the 5' end only in the nucleotide sequence represented by SEQ ID NO:22, 24, 26, 28, 30, 32, 34 or 36. In the case that the nucleotide sequence 1fa satisfies the relation of the (A) with the nucleotide sequence represented by SEQ ID NO:22, 24, 26, 28, 30, 32, 34 or 36, it is more preferred that the nucleotide sequence 1fa is a sequence in which one or several bases in total are added to either or both of the 5' end and the 3' end in the nucleotide sequence represented by SEQ ID NO:22, 24, 26, 28, 30, 32, 34 or 36, and it is particularly preferred that the nucleotide sequence 1fa is a partial nucleotide sequence of the nucleotide sequence represented by SEQ ID NO:17, wherein the partial nucleotide sequence consists of the nucleotide sequence represented by SEQ ID NO:22, 24, 26, 28, 30, 32, 34 or 36 and one or several bases in total added to either or both of the 5' end and the 3' end of the nucleotide sequence represented by SEQ ID NO:22, 24, 26, 28, 30, 32, 34 or 36.

Other preferred examples of the cases that the nucleotide sequence 1fa satisfies the relation of the (A) with the nucleotide sequence represented by SEQ ID NO:22, 24, 26, 28, 30, 32, 34 or 36 include, for example, the following embodiment. The partial nucleotide sequences corresponding to the nucleotide sequence represented by SEQ ID NOS:22, 24, 26, 28, 30, 32, 34 and 36 in the nucleotide sequence represented by SEQ ID NO:18, 19, 20 or 21, are set as partial nucleotide sequences 22', 24', 26', 28', 30', 32', 34' and 36', respectively, upon aligning the nucleotide sequence represented by SEQ ID NO:18, 19, 20 or 21 with the nucleotide sequence represented by SEQ ID NO:17 so as to maximize the matching degree of the nucleotide sequence in the specific region 1. When the partial nucleotide sequence 22', 24', 26', 28', 30', 32', 34' or 36' is a nucleotide sequence containing one or more modifications in which one or several bases are deleted, substituted, added and/or inserted in the nucleotide sequence represented by SEQ ID NO:22, 24, 26, 28, 30, 32, 34 or 36, respectively, a nucleotide sequence in which at least one of the modifications (deletion, substitution, addition and/or insertion) are introduced into the nucleotide sequence represented by SEQ ID NO:22, 24, 26, 28, 30, 32, 34 or 36 can be set as nucleotide sequence 1fa, and more preferably, the partial nucleotide sequence 22', 24', 26', 28', 30', 32', 34' or 36' can be set as nucleotide sequence 1fa.

In the (1f), it is particularly preferred that nucleotide sequence 1fa is identical to the nucleotide sequence represented by SEQ ID NO:22, 24, 26, 28, 30, 32, 34 or 36.

In the (1f), the nucleotide sequence 1fb is a nucleotide sequence contained in the nucleotide sequence 1fa having consecutive 8 or more bases, preferably 10 or more bases, more preferably 12 or more bases, more preferably 15 or more bases, and the nucleotide sequence 1fb may be identical to the nucleotide sequence 1fa or may be a partial nucleotide sequence of the nucleotide sequence 1fa. The nucleotide sequence 1fb is preferably a consecutive nucleotide sequence comprising a base of the 3' end contained in the nucleotide sequence 1fa.

In the (1f), it is particularly preferred that nucleotide sequence 1fb is identical to the nucleotide sequence 1fa.

The polynucleotide of (1f) comprises the nucleotide sequence 1fb at the 3' end, and it may further comprise another nucleotide sequence at the 5' end side of the nucleotide sequence 1fb. The polynucleotide of (1f) is a polynucleotide having 40 or less bases, preferably 35 or less bases, more preferably 30 or less bases, more preferably 25 or less bases, particularly preferably 22 or less bases.

It is particularly preferred that the polynucleotide of (1f) consists of the nucleotide sequence 1fb.

In the (1r), the nucleotide sequence 1ra is identical to the nucleotide sequence represented by SEQ ID NO:23, 25, 27, 29, 31, 33, 35 or 37, in other words, satisfies the relation of the (A), (B), (C) or (D) (the nucleotide sequence represented by SEQ ID NO:23, 25, 27, 29, 31, 33, 35 or 37 corresponds to the nucleotide sequence X, and the nucleotide sequence 1ra corresponds to the nucleotide sequence Y).

In the case that the nucleotide sequence 1ra satisfies the relation of the (A) with the nucleotide sequence represented by SEQ ID NO:23, 25, 27, 29, 31, 33, 35 or 37, it is more preferred that the nucleotide sequence 1ra is a sequence in which one or several bases in total are deleted from either or both of the 5' end and the 3' end in the nucleotide sequence represented by SEQ ID NO:23, 25, 27, 29, 31, 33, 35 or 37, and it is particularly preferred that the nucleotide sequence 1ra is a sequence in which one or several bases in total are deleted from the 5' end only in the nucleotide sequence represented by SEQ ID NO:23, 25, 27, 29, 31, 33, 35 or 37. In the case that the nucleotide sequence 1ra satisfies the relation of the (A) with the nucleotide sequence represented by SEQ ID NO:23, 25, 27, 29, 31, 33, 35 or 37, it is more preferred that the nucleotide sequence 1ra is a sequence in which one or several bases in total are added to either or both of the 5' end and the 3' end in the nucleotide sequence represented by SEQ ID NO:23, 25, 27, 29, 31, 33, 35 or 37, and it is particularly preferred that the nucleotide sequence 1ra is a partial nucleotide sequence of a complementary nucleotide sequence to the nucleotide sequence represented by SEQ ID NO:17, wherein the partial nucleotide sequence consists of the nucleotide sequence represented by SEQ ID NO:23, 25, 27, 29, 31, 33, 35 or 37 and one or several bases in total added to either or both of the 5' end and the 3' end of the nucleotide sequence represented by SEQ ID NO:23, 25, 27, 29, 31, 33, 35 or 37.

Other preferred examples of the cases that the nucleotide sequence 1ra satisfies the relation of the (A) with the nucleotide sequence represented by SEQ ID NO:23, 25, 27, 29, 31, 33, 35 or 37 include, for example, the following embodiment. The partial nucleotide sequences corresponding to the nucleotide sequence represented by SEQ ID NOS:23, 25, 27, 29, 31, 33, 35 and 37 in the complementary nucleotide sequence to the nucleotide sequence represented by SEQ ID NO:18, 19, 20 or 21, are set as partial nucleotide sequences 23', 25', 27', 29', 31', 33', 35' and 37', respectively, upon aligning the complementary nucleotide sequence to the nucleotide sequence represented by SEQ ID NO:18, 19, 20 or 21 with the complementary nucleotide sequence to the nucleotide sequence represented by SEQ ID NO:17 so as to maximize the matching degree of the complementary nucleotide sequence in the specific region 1. When the partial nucleotide sequence 23', 25', 27', 29', 31', 33', 35' or 37' is a nucleotide sequence containing one or more modifications in which one or several bases are deleted, substituted, added and/or inserted in the nucleotide sequence represented by SEQ ID NO:23, 25, 27, 29, 31, 33, 35 or 37, respectively, a nucleotide sequence in which at least one of the modifications (deletion, substitution, addition and/or insertion) are introduced into the nucleotide sequence represented by SEQ ID NO:23, 25, 27, 29, 31, 33, 35 or 37 can be set as nucleotide sequence 1ra, and more preferably, the partial nucleotide sequence 23', 25', 27', 29', 31', 33', 35' or 37' can be set as nucleotide sequence 1ra.

In the (1r), it is particularly preferred that the nucleotide sequence 1ra is identical to the nucleotide sequence represented by SEQ ID NO:23, 25, 27, 29, 31, 33, 35 or 37.

In the (1r), the nucleotide sequence 1rb is a nucleotide sequence contained in the nucleotide sequence 1ra having consecutive 8 or more bases, preferably 10 or more bases, more preferably 12 or more bases, more preferably 15 or more bases, and the nucleotide sequence 1rb may be identical to the nucleotide sequence 1ra or may be a partial nucleotide sequence of the nucleotide sequence 1ra. The nucleotide sequence 1rb is preferably a consecutive nucleotide sequence comprising a base of the 3' end contained in the nucleotide sequence 1ra.

In the (1r), it is particularly preferred that the nucleotide sequence 1rb is identical to the nucleotide sequence 1ra.

The polynucleotide of (1r) comprises the nucleotide sequence 1rb at the 3' end, and it may further comprise another nucleotide sequence at the 5' end side of the nucleotide sequence 1rb. The polynucleotide of (1r) is a polynucleotide having 40 or less bases, preferably 35 or less bases, more preferably 30 or less bases, more preferably 25 or less bases, particularly preferably 22 or less bases.

It is particularly preferred that the polynucleotide of (1r) consists of the nucleotide sequence 1rb.

The combination of the first primer comprising the polynucleotide of (1f) and the second primer comprising the polynucleotide of (1r) is not particularly limited, and they can be combined to allow the target region of the genomic DNA to be amplified as a polynucleotide fragment. Specific examples of the combination include the following.

When the "nucleotide sequence represented by SEQ ID NO:22, 24, 26, 28, 30, 32, 34 or 36" in the (1f) is "nucleotide sequence represented by SEQ ID NO:22", "nucleotide sequence represented by SEQ ID NO:23, 25, 27, 29, 31, 33, 35 or 37" in the (1r) may be any of them, but preferably "nucleotide sequence represented by SEQ ID NO:23". In particular, it is preferably a combination of the polynucleotide of (1f) which is a polynucleotide consisting of a nucleotide sequence represented by SEQ ID NO:22 (MKS-40f) and the polynucleotide of (1r) which is a polynucleotide consisting of a nucleotide sequence represented by SEQ ID NO:23 (MKS-44r), the combination being "primer set 4".

When "nucleotide sequence represented by SEQ ID NO:22, 24, 26, 28, 30, 32, 34 or 36" in the (1f) is "nucleotide sequence represented by SEQ ID NO:24", "nucleotide sequence represented by SEQ ID NO:23, 25, 27, 29, 31, 33, 35 or 37" in the (1r) may be any of them, but preferably "nucleotide sequence represented by SEQ ID NO:25". In particular, it is preferably a combination of the polynucleotide of (1f) which is a polynucleotide consisting of a nucleotide sequence represented by SEQ ID NO:24 (MKS-41f) and the polynucleotide of (1r) which is a polynucleotide consisting of a nucleotide sequence represented by SEQ ID NO:25 (MKS-41r), the combination being "primer set 5".

When "nucleotide sequence represented by SEQ ID NO:22, 24, 26, 28, 30, 32, 34 or 36" in the (1f) is "nucleotide sequence represented by SEQ ID NO:26", "nucleotide sequence represented by SEQ ID NO:23, 25, 27, 29, 31, 33, 35 or 37" in the (1r) may be any of them, but preferably "nucleotide sequence represented by SEQ ID NO:27". In particular, it is preferably a combination of the polynucleotide of (1f) which is a polynucleotide consisting of a nucleotide sequence represented by SEQ ID NO:26 (MKS-42f) and the polynucleotide of (1r) which is a polynucleotide consisting of a nucleotide sequence represented by SEQ ID NO:27 (MKS-42r), the combination being "primer set 6".

When "nucleotide sequence represented by SEQ ID NO:22, 24, 26, 28, 30, 32, 34 or 36" in the (1f) is "nucleotide sequence represented by SEQ ID NO:28", "nucleotide sequence represented by SEQ ID NO:23, 25, 27, 29, 31, 33, 35 or 37" in the (1r) is "nucleotide sequence represented by SEQ ID NO:23, 25, 29, 35 or 37", and preferably "nucleotide sequence represented by SEQ ID NO:29". In particular, it is preferably a combination of the polynucleotide of (1f) which is a polynucleotide consisting of a nucleotide sequence represented by SEQ ID NO:28 (MKS-43f) and the polynucleotide of (1r) which is a polynucleotide consisting of a nucleotide sequence represented by SEQ ID NO:29 (MKS-43r), the combination being "primer set 7".

When "nucleotide sequence represented by SEQ ID NO:22, 24, 26, 28, 30, 32, 34 or 36" in the (1f) is "nucleotide sequence represented by SEQ ID NO:30", "nucleotide sequence represented by SEQ ID NO:23, 25, 27, 29, 31, 33, 35 or 37" in the (1r) may be any of them, but preferably "nucleotide sequence represented by SEQ ID NO:31". In particular, it is preferred that it is a combination of the polynucleotide of (1f) which is a polynucleotide consisting of a nucleotide sequence represented by SEQ ID NO:30 (MKS-44f) and the polynucleotide of (1r) which is a polynucleotide consisting of a nucleotide sequence represented by SEQ ID NO:31 (MKS-44r), the combination being "primer set 8".

When "nucleotide sequence represented by SEQ ID NO:22, 24, 26, 28, 30, 32, 34 or 36" in the (1f) is "nucleotide sequence represented by SEQ ID NO:32", "nucleotide sequence represented by SEQ ID NO:23, 25, 27, 29, 31, 33, 35 or 37" in the (1r) may be any of them, but preferably "nucleotide sequence represented by SEQ ID NO:33". In particular, it is preferably a combination of the polynucleotide of (1f) which is a polynucleotide consisting of a nucleotide sequence represented by SEQ ID NO:32 (MKS-45f) and the polynucleotide of (1r) which is a polynucleotide consisting of a nucleotide sequence represented by SEQ ID NO:33 (MKS-45r), the combination being "primer set 9".

When "nucleotide sequence represented by SEQ ID NO:22, 24, 26, 28, 30, 32, 34 or 36" in the (1f) is "nucleotide sequence represented by SEQ ID NO:34", "nucleotide sequence represented by SEQ ID NO:23, 25, 27, 29, 31, 33, 35 or 37" in the (1r) is "nucleotide sequence represented by SEQ ID NO:23, 25, 29, 35 or 37", and preferably "nucleotide sequence represented by SEQ ID NO:35". In particular, it is preferably a combination of the polynucleotide of (1f) which is a polynucleotide consisting of a nucleotide sequence represented by SEQ ID NO:34 (MKS-46f) and the polynucleotide of (1r) which is a polynucleotide consisting of a nucleotide sequence represented by SEQ ID NO:35 (MKS-46r), the combination being "primer set 10".

When "nucleotide sequence represented by SEQ ID NO:22, 24, 26, 28, 30, 32, 34 or 36" in the (1f) is "nucleotide sequence represented by SEQ ID NO:36", "nucleotide sequence represented by SEQ ID NO:23, 25, 27, 29, 31, 33, 35 or 37" in the (1r) is "nucleotide sequence represented by SEQ ID NO:23, 25, 29, 35 or 37", and preferably "nucleotide sequence represented by SEQ ID NO:37". In particular, it is preferably a combination of the polynucleotide of (1f) which is a polynucleotide consisting of a nucleotide sequence represented by SEQ ID NO:36 (MKS-47f) and the polynucleotide of (1r) which is a polynucleotide consisting of a nucleotide sequence represented by SEQ ID NO:37 (MKS-47r), the combination being "primer set 11".

**[Table 2]**

| | Primer | Sequence (5'→3') | SEQ ID NO: |
|---|---|---|---|
| Primer set 4 | MKS-40f | ACGAGTGAGAACGAGGAGGA | 22 |
| | MKS-40r | ATGTTCGGCATACCACCGTT | 23 |
| Primer set 5 | MKS-41f | TGAGAACGAGGAGGAGCCAT | 24 |
| | MKS-41r | TGTTCGGCATACCACCGTTC | 25 |
| Primer set 6 | MKS-42f | CTGACGAGTGAGAACGAGGAG | 26 |
| | MKS-42r | GATCCGACACCGCCGATAA | 27 |
| Primer set 7 | MKS-43f | TTATCGGCGGTGTCGGATCT | 28 |
| | MKS-43r | TTCGGCATACCACCGTTCG | 29 |
| Primer set 8 | MKS-44f | TCGCTGACGAGTGAGAACG | 30 |
| | MKS-44r | GAGATCCGACACCGCCGATA | 31 |
| Primer set 9 | MKS-45f | TCGCTGACGAGTGAGAACGA | 32 |
| | MKS-45r | GTATGCAGGTGGTCGACGG | 33 |
| Primer set 10 | MKS-46f | TATCGGCGGTGTCGGATCTC | 34 |
| | MKS-46r | GTTCGGCATACCACCGTTCG | 35 |
| Primer set 11 | MKS-47f | ATGACTCCCAGGGCATGGT | 36 |
| | MKS-47r | GATGTTCGGCATACCACCGTT | 37 |

The first primer may consist of only the polynucleotide described above, or may comprise, in addition to the polynucleotide, a label portion or a binding portion described below. The polynucleotide and the label portion or binding portion can be chemically linked via an appropriate spacer described below. In the first primer, the position of one of the label portion and the binding portion is linked to the polynucleotide is not particularly limited as long as the position does not inhibit the elongation in the annealing and nucleic acid amplification reaction of the polynucleotide with a polynucleotide comprising a target region or a complementary strand thereof, but it is preferably the 5' end of the polynucleotide.

The second primer may consist of only the polynucleotide described above, or may comprise, in addition to the polynucleotide, a label portion or a binding portion described below. The polynucleotide and the label portion or binding portion can be chemically linked via an appropriate spacer described below. In the second primer, the position of one of the label portion and the binding portion is linked to the polynucleotide is not particularly limited as long as the position does not inhibit the elongation in the annealing and nucleic acid amplification reaction of the polynucleotide with a polynucleotide comprising a target region or a complementary strand thereof, but it is preferably the 5' end of the polynucleotide.

When at least one of the first primer and the second primer comprise a label portion, the detection of amplified products is easy because a nucleic acid amplification reaction using such a primer yields amplified products comprising the label portion.

When at least one of the first primer and the second primer comprise a binding portion, the detection of amplified products is easy because a nucleic acid amplification reaction using such a primer yields amplified products comprising the binding portion which can be captured to a solid phase carrier.

More preferably, one of the first primer and the second primer further comprises a label portion, and the other further comprises a binding portion. When using a pair of primers of this embodiment in a nucleic acid amplification reaction, the detection by nucleic acid chromatography is easy because the nucleic acid amplification reaction yields amplified products of double strand comprising the label portion and the binding portion. Hereinafter, this embodiment will be described.

### (Preferred Embodiment of Primer Set of The Present Invention)

### (Label Portion)

The label portion is either a label substance or a tag capable of binding to a label substance, and preferably a tag capable of binding to a label substance. In this description, the tag capable of binding to a label substance is sometimes referred to as a labelling tag. When the label portion is a labelling tag, an amplified product obtained by a nucleic acid amplification reaction can be contacted with a label substance to label the tag contained in an end of the amplified product. When the label portion is a label substance, an amplified product containing a label substance in an end can be obtained as the amplified product of the nucleic acid amplification reaction.

The label substance is not particularly limited as long as that makes the amplified product detectable, but it is preferably a substance that makes the amplified product visually detectable. Examples of such a label substance include a colored particle, a dye, an enzyme (peroxidase, alkaline phosphatase, luciferase, and the like) and it is preferably a colored particle. Examples of the "colored particle" include, but are not limited to, a metal (for example, gold, silver, copper, and platinum) particle, a metal rod, a colored latex particle, and a dye-containing silica nanoparticle. The size of the label substance is not limited as long as the size does not prevent the trapping of the amplified product to a solid phase carrier described below. It is preferred that the label substances have good color development upon detection. The size of the label substance can be selected appropriately so as to be smaller than the pore size of a solid phase carrier described below or various porous member of the nucleic acid detection device comprising a solid support. For example, the size of label substance may be about 500 nm or less, preferably about 0.1 nm to 250 nm, more preferably about 1 nm to 100 nm in particle diameter. As the dye, fluorescent dyes (cyanine, fluorescein, and the like) can be used, and in that case, it is preferred to irradiate with excitation wavelength light of each fluorescent dye to detect.

The labelling tag usable as the label portion is not particularly limited as long as it is capable of binding to a label substance, and can be selected appropriately depending on the structure of the label substance. Examples of such a tag include a nucleic acid (DNA, RNA, or the like), a protein, a peptide or other compound (e.g., biotin, fluorescein isothiocyanate (FITC), low molecular weight compound such as digoxigenin (DIG)), and a combination of these. In one preferred embodiment, the labelling tag comprises or consists of a polynucleotide. The polynucleotide which may be included in the labelling tag is not particularly limited as long as it does not substantially inhibit the nucleic acid amplification reaction by the primer set of the present invention, but it may be a polynucleotide having, for example, 5 to 50 bases, preferably 10 to 35 bases, and more preferably it may be a polynucleotide comprising (or consisting of) a nucleotide sequence represented by SEQ ID NO:41 or 42 or a partial nucleotide sequence thereof or their complementary nucleotide sequence. In another preferred embodiment of the labelling tag, the labelling tag consists of a low molecular compound such as biotin, FITC, and DIG.

When a label portion is the labelling tag described above, a label substance may be directly or indirectly bound to the labelling tag, and a suitable binding means can be selected appropriately depending on the combination of the label substance and the labelling tag to be used. For example, when the labelling tag comprises a polynucleotide, a label substance may be indirectly bound to the labelling tag by binding the label substance to a polynucleotide capable of hybridizing to the polynucleotide (for example, a polynucleotide comprising a sequence complementary to the nucleotide sequence of the polynucleotide), and causing hybridization between the polynucleotides. Binding of the label substance to the polynucleotide may be performed via a peptide, a protein, a nucleic acid or the like, and may be performed via a suitable functional group. Conditions of hybridization are not particularly limited as long as it causes hybridization, but, for example, a hybridization can occur by performing a reaction at 20 to 40°C in a buffer containing 10 to 50 mM phosphate (pH 6 to 7). To enhance the efficiency of hybridization, the buffer may further contain a salt such as sodium chloride.

When the labelling tag is a low molecular weight compound, labelling can be performed with a label substance coupled to an binding agent such as a protein specifically bind to a compound (such as avidin that binds to biotin, a protein that binds to FITC), an antibody (e.g., anti-DIG antibody), and an aptamer. In this case, the labelling tag can be bound to the binding substance using various buffers near neutral.

The label portion (labelling tag or label substance) can be bound to the polynucleotides contained in the first primer or the polynucleotides contained in the second primer, via any means, and directly or indirectly. However, when a part in the label portion connecting to at least the polynucleotide is composed of a polynucleotide, the polynucleotide and the label portion are bound via a spacer capable of suppressing or halting a progression of a DNA polymerase reaction so as not to form a double strand of the part and the polynucleotide due to a nucleic acid amplification reaction. Such a "spacer" is not limited as long as it can suppress or halt the progression of a DNA polymerase reaction and prevents the label portion being double stranded. Examples of such a "spacer" include, but not limited to, a nucleic acid sequence having a strong hairpin structure and/or a pseudoknot structure, an L-type nucleic acid, a peptide nucleic acid (PNA), a cross-linked nucleic acid (bridged nucleic acid (BNA) or locked nucleic acid (LNA)), fluorescein, Cy3, Cy5, a divalent group containing an azobenzene structure represented by the following formula I, aliphatic chain (alkylene chain or polyoxyalkylene chain), a divalent group containing inverted array structure such as 5'-5' and 3'-3' linkages.

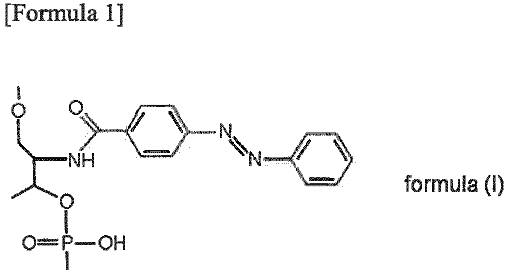

When connecting two polynucleotide molecules via a divalent group represented by formula I, the phosphate group of one end of the divalent group refers to a phosphate group of a nucleotide at the 3' end or 5' end of one polynucleotide molecule, and an oxygen atom of the other end, together with the phosphate group of a nucleotide at the 3' end or 5' end of the other polynucleotide molecule, forms a phosphoric acid ester bond.

Examples of the fatty chain spacer include a spacer represented by the following formula (II).

-O-CₘH₂ₘ-O- formula (II)

(wherein, the oxygen atom at one end represents an oxygen atom in a phosphoric acid diester bond at the 3' end or 5' end of one polynucleotide molecule, and the oxygen atom of the other end represents an oxygen atom in a phosphate diester bond at the 3' end or 5' end of the other polynucleotide molecule, m represents an integer of 1 or more and 40 or less. H may be substituted by a substituent.)

In formula (II), m is preferably 2 or more and 36 or less, more preferably 3 or more and 16 or less. H in formula (II) may be substituted by a substituent, and examples of the substituent typically include an alkyl group, an alkoxy group, and a hydroxyl group. The number of carbon atoms in the alkyl group and alkoxy group as the substituent is preferably from 1 to 8, more preferably 1 to 4. When there are 2 or more substituents, the substituents may be the same or different. The spacer of formula (II) with no substituents is also preferred.

Other examples of the spacer include a spacer represented by the following formula (III).

-(OCₙH₂ₙ)_{L}-O- formula (III)

(wherein, the oxygen atom at one end represents an oxygen atom in a phosphoric acid diester bond at the 3' end or 5' end of one polynucleotide molecule, and the oxygen atom of the other end represents an oxygen atom in a phosphate diester bond at the 3' end or 5' end of the other polynucleotide molecule, n represents an integer of 2 or more and 4 or less, L is an integer of 1 or more, and (n + 1) × L is an integer of 40 or less. H may be substituted by a substituent.)

In formula (III), (n + 1) × L is preferably 2 or more and 36 or less, more preferably 3 or more and 16 or less. Substituents of H in formula (III) are as in the substituents in formula (II).

Other examples of fatty chain spacers include the following divalent groups.

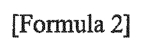

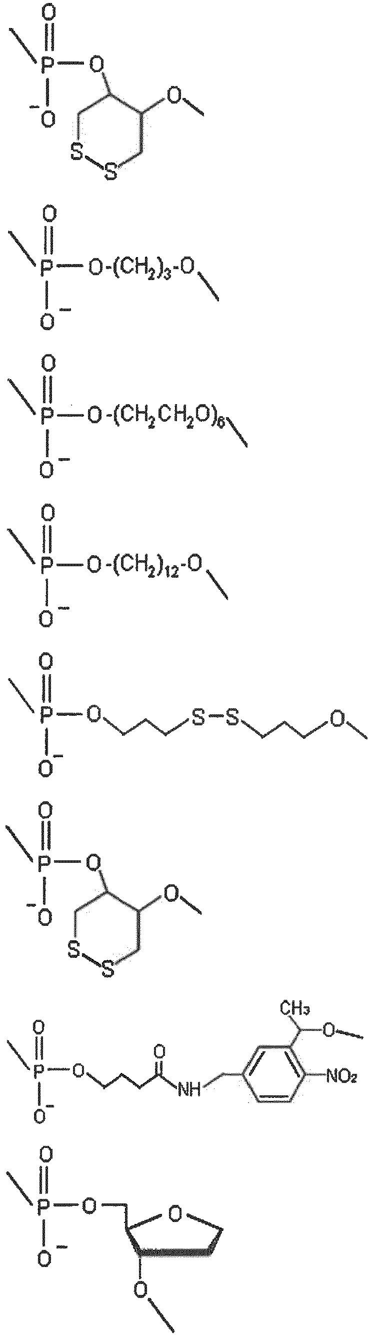

When connecting two polynucleotide molecules via these divalent groups, the phosphate group of one end of each divalent group refers to a phosphate group of a nucleotide at the 3' end or 5' end of one polynucleotide molecule, and an oxygen atom of the other end, together with the phosphate group of a nucleotide at the 3' end or 5' end of the other polynucleotide molecule, forms a phosphoric acid ester bond.

### (Binding Portion and Solid phase carrier)

The binding portion is a tag capable of binding to a solid phase carrier described below. In this description, the tag capable of binding to a solid phase carrier is sometimes referred to as a tag for binding.

The tag for binding which can be used as the binding portion is not particularly limited as long as it is capable of binding to a solid phase carrier, and can be selected appropriately depending on the structure of the solid phase carrier. Examples of such a tag for binding include a polynucleotide (DNA, RNA, or the like), a protein, a peptide or other compound (e.g., a low molecular weight compound) and a combination of these. In one preferred embodiment, the tag for binding comprises or consists of a polynucleotide. The polynucleotide which may be comprised in a tag for binding is not particularly limited as long as it does not substantially inhibit the nucleic acid amplification reaction by the primer set of the present invention, but it may be a polynucleotide having, for example, 5 to 50 bases, preferably 10 to 35 bases, and more preferably it may be a polynucleotide comprising (or consisting of) a nucleotide sequence represented by SEQ ID NO:41 or 42 or a partial nucleotide sequence thereof or their complementary nucleotide sequence.

The solid phase carrier is not particularly limited, and it may be made of resin, metal, polysaccharide, mineral or the like, and it may be in the form of a membrane, a film, a nonwoven fabric, a plate, a gel, and the like. Preferably, the solid phase carrier is one having a porous structure so as to allow the development of the amplification product and/or the label substance in the solution. Examples of the solid phase carrier usable in the present invention include a filter paper, a nitrocellulose membrane, a polyethersulfone membrane, a nylon membrane, dried various gel (silica gel, agarose gel, dextran gel, gelatin gel), silicon, glass, and plastic. The size and shape of the solid phase carrier can be selected appropriately so as to be suitable for various operations and detection.

The solid phase carrier is configured to allow binding of at least a part of the solid phase carrier to the tag for binding, and it is more preferred that it is configured to allow binding of only a part of the solid phase carrier to the tag for binding. By binding only a certain part of the solid phase carrier to the tag for binding, positive or negative determination can be facilitated since the amplification product captured by the solid phase carrier is detected only at the certain part restrictively.

The solid phase carrier may bind directly or indirectly to the tag for binding, and a suitable binding means can be selected appropriately depending on the combination of the solid phase carrier and the tag for binding to be used. For example, when the tag for binding comprises a polynucleotide, a solid phase carrier may be indirectly bound to the tag for binding by immobilizing a polynucleotide capable of hybridizing to the polynucleotide (for example, a polynucleotide comprising a sequence complementary to the nucleotide sequence of the polynucleotide) on the solid phase carrier to provide a tag capture means, and causing hybridization between the two polynucleotides. Immobilization of the polynucleotide on the solid phase carrier may be carried out via a peptide, a protein, a nucleic acid or other means, and may be carried out via a suitable functional group. Hybridization can be performed by the condition as described above with respect to the binding of the labelling tag to the label substance. When the polynucleotide is immobilized only to a certain part of a solid phase carrier, positive or negative determination can be facilitated since the captured amplification product is detected restrictively only to the certain part.

The binding portion (tag for binding) can bind to a polynucleotide contained in the first primer or a polynucleotide contained in the second primer by any means, and directly or indirectly. However, when at least a part connecting to the polynucleotide in the tag for binding is composed of a nucleic acid, the polynucleotide and the tag for binding are bound via a spacer capable of suppressing or halting a progression of a DNA polymerase reaction so as not to form a double strand of the part and the polynucleotide by a nucleic acid amplification reaction. Specific examples of the spacer provided between the binding portion and the polynucleotide are the same as ones described above with respect to the spacers provided between the label portion and the polynucleotide.

### <4. Probe>

The present invention also relates to a probe as defined in claim 3.

The probe of the present invention can be hybridized with a polynucleotide comprising the first nucleotide sequence or the second nucleotide sequence; or a third nucleotide sequence comprising the first nucleotide sequence or second nucleotide sequence in which any T is replaced with U under a stringent condition. Therefore, the probe of the present invention can be used in nucleic acid hybridization methods (e.g., Southern hybridization), real-time PCR methods (e.g. TaqMan(TM) method, Molecular Beacon method), and the like.

The polynucleotide in the probe of the present invention comprises a nucleotide sequence identical or homologous to a partial nucleotide sequence of the first nucleotide sequence or the second nucleotide sequence (hereinafter referred to as "partial nucleotide sequence P").

The partial nucleotide sequence P is a nucleotide sequence in the consecutive part of the first nucleotide sequence or the second nucleotide sequence. The number of bases must be sufficient to keep the specificity, and the partial nucleotide sequence has 15 or more bases, more preferably 20 or more bases in the nucleotide sequence represented by SEQ ID NO:38, 39 or 40 or a complementary sequence thereof. The upper limit of the base numbers of the partial nucleotide sequence P is not particularly limited, but, for example, the partial nucleotide sequence P can be a partial nucleotide sequence having consecutive 400 or less bases, 300 or less bases, or 200 or less bases in the first nucleotide sequence or the second nucleotide sequence. The polynucleotides in the probe of the present invention is not required to consist of only a nucleotide sequence identical or homologous to the partial nucleotide sequence P (hereinafter referred to as "nucleotide sequence Q"), it may further comprise a further nucleotide sequence at the 3' end side and/or the 5' end side of the nucleotide sequence Q. It is particularly preferred that the nucleotide sequence Q is identical to the partial nucleotide sequence P.

Total number of bases of the polynucleotide in the probe of the present invention is not particularly limited, but it is 15 or more bases, more preferably 20 or more bases, and can be preferably 400 or less bases, 300 or less bases, or 200 or less bases. When using the probe of the present invention in a real time PCR method, the polynucleotide is preferably 50 or less bases, more preferably 40 or less bases, or 35 or less bases.

SEQ ID NO:38 corresponds to position 113 to position 139 of SEQ ID NO:2 and position 3644 to position 3670 of SEQ ID NO:17.

SEQ ID NO:39 corresponds to position 262 to position 292 of SEQ ID NO:2 and position 3793 to position 3823 of SEQ ID NO:17.

SEQ ID NO:40 corresponds to position 435 to position 464 of SEQ ID NO:2 and position 3966 to position 3995 of SEQ ID NO:17.

In the (2p), the nucleotide sequence 2pa is identical to the nucleotide sequence represented by SEQ ID NO:38, 39 or 40 or a complementary nucleotide sequence thereof, or is homologous to them, in other words, satisfies the relation of the (A), (B), (C) or (D) (the nucleotide sequence represented by SEQ ID NO:38, 39 or 40 or a complementary nucleotide sequence thereof corresponds to the nucleotide sequence X, and the nucleotide sequence 2pa corresponds to the nucleotide sequence Y).

In the case that the nucleotide sequence 2pa satisfies the relation of the (A) with the nucleotide sequence represented by SEQ ID NO:38, 39 or 40 or a complementary nucleotide sequence thereof, it is more preferred that the nucleotide sequence 2pa is a sequence in which one or several bases in total are deleted from either or both of the 5' end and the 3' end in the nucleotide sequence represented by SEQ ID NO:38, 39 or 40 or a complementary nucleotide sequence thereof. In the case that the nucleotide sequence 2pa satisfies the relation of the (A) with the nucleotide sequence represented by SEQ ID NO:38, 39 or 40 or a complementary nucleotide sequence thereof, it is more preferred that the nucleotide sequence 2pa is a sequence in which one or several bases in total are added to either or both of the 5' end and the 3' end in the nucleotide sequence represented by SEQ ID NO:38, 39 or 40 or a complementary nucleotide sequence thereof, and it is particularly preferred that the nucleotide sequence 2pa is a partial nucleotide sequence of the nucleotide sequence represented by SEQ ID NO:17 or a complementary nucleotide sequence thereof, wherein the partial nucleotide sequence consists of the nucleotide sequence represented by SEQ ID NO:38, 39 or 40 or a complementary nucleotide sequence thereof and one or several bases in total added to either or both of the 5' end and the 3' end of the nucleotide sequence represented by SEQ ID NO:38, 39 or 40 or the complementary nucleotide sequence thereof.

Other preferred examples of the cases that the nucleotide sequence 2pa satisfies the relation of the (A) with the nucleotide sequence represented by SEQ ID NO:38, 39 or 40 include, for example, the following embodiment. The partial nucleotide sequences corresponding to the nucleotide sequence represented by SEQ ID NOS: 38, 39 and 40 in the nucleotide sequence represented by SEQ ID NO:18, 19, 20 or 21, are set as partial nucleotide sequences represented by SEQ ID NOS:38', 39' and 40', respectively, upon aligning the nucleotide sequence represented by SEQ ID NO: 18, 19, 20 or 21 with the nucleotide sequence represented by SEQ ID NO: 17 so as to maximize the matching degree of the nucleotide sequence in the specific region 2. When the partial nucleotide sequence 38', 39' or 40' is a nucleotide sequence containing one or more modifications in which one or several bases are deleted, substituted, added and/or inserted in the nucleotide sequence represented by SEQ ID NO:38, 39 or 40, respectively, a nucleotide sequence in which at least one of the modifications (deletion, substitution, addition and/or insertion) are introduced into the nucleotide sequence represented by SEQ ID NO:38, 39 or 40 or a complementary nucleotide sequence thereof can be set as nucleotide sequence 2pa, and more preferably, the partial nucleotide sequence 38', 39' or 40' or a complementary nucleotide sequence thereof can be set as nucleotide sequence 2pa.

In the (2p), it is particularly preferred that the nucleotide sequence 2pa is identical to the nucleotide sequence represented by SEQ ID NO:38, 39 or 40 or a complementary nucleotide sequence thereof.

In the (2p), the nucleotide sequence 2pb is a nucleotide sequence having consecutive 8 or more bases, preferably 10 or more bases, more preferably 12 or more bases, more preferably 15 or more bases, more preferably 20 or more bases, more preferably 25 or more bases contained in the nucleotide sequence 2pa, and may be identical to the nucleotide sequence 2pa or may be a partial nucleotide sequence of nucleotide sequence 2pa. The nucleotide sequence 2pb is preferably a consecutive nucleotide sequence comprising a base of the 3' end contained in the nucleotide sequence 2pa.

In the (2p), it is particularly preferred that the nucleotide sequence 2pb is identical to the nucleotide sequence 2pa.

The polynucleotide of (2p) comprises the nucleotide sequence 2pb, and it may further comprise another nucleotide sequence at the 3' end side and/or the 5' end side of the nucleotide sequence 2pb. Total number of bases of the polynucleotide of (2p) is not particularly limited, but it is preferably 100 or less bases or 50 or less bases, more preferably 40 or less bases or 35 or less bases.

It is particularly preferred that the polynucleotide of (2P) consists of nucleotide sequence 2pb.

Specific examples of the polynucleotide of (2p) include a polynucleotide consisting of a nucleotide sequence represented by SEQ ID NO:38, 39 or 40 or a complementary nucleotide sequence thereof.

The probes of the present invention may further comprise, besides the polynucleotide described above, a label portion which is a label substance or a tag capable of binding to a label substance. Specific examples of such a label portion is as described above with respect to the primer. A complex generated by hybridizing the probe of the present invention comprising the label portion with a polynucleotide comprising the first nucleotide sequence, the second nucleotide sequence or the third nucleotide sequence is easy to detect by using the label portion as an indicator.

The probes of the present invention may also further comprise, besides the polynucleotide described above, a binding portion which is a tag capable of binding to a solid phase carrier. Examples of such a binding portion are as described above with respect to the primer. A complex generated by hybridizing the probe of the present invention comprising the binding portion with a polynucleotide comprising the first nucleotide sequence, the second nucleotide sequence or the third nucleotide sequence is easy to detect and isolate the complex since the complex can be bound to a solid phase carrier via the binding portion.

The probe of the present invention comprising the binding portion may be immobilized on a solid phase carrier in advance via the binding portion. The probe of the present invention immobilized on a solid phase carrier is able to capture a polynucleotide comprising the first nucleotide sequence, the second nucleotide sequence or the third nucleotide sequence.

It is preferable that the probe of the present invention, when used in a real-time PCR method, be labeled with a label substance normally used in a real-time detection of amplification products. For example, the 5' end of the polynucleotide is linked to a reporter fluorescent substance, and the 3' end thereof is linked to a quencher dye. Examples of the reporter fluorescent substance include carboxyfluorescein (FAM), hexachlorofluorescein (HEX), and tetrachlorofluorescein (TET). Examples of the quencher dye include fluorescent substances such as carboxytetramethylrhodamine (TAMRA), and non-fluorescent substances such as Black Hole Quencher dye (BHQ), and 4-((4-(dimethylamino)phenyl)azo)benzoic acid (DABCYL).

Specific examples of the probe of the present invention for use in a real-time PCR method include a probe comprising the polynucleotide of (2P) to which a reporter fluorescent substance is linked at the 5' end and a quencher dye is linked at the 3' end. A probe for a real-time PCR method, comprising a polynucleotide which is "nucleotide sequence represented by SEQ ID NO:38" in the "nucleotide sequence represented by SEQ ID NO:38, 39 or 40" of the (2p) is suitable for performing real-time detection of amplified products in PCR using primer set 2. A probe for a real-time PCR method comprising a polynucleotide which is "nucleotide sequence represented by SEQ ID NO:39" in the "nucleotide sequence represented by SEQ ID NO:38, 39 or 40" of the (2p) is suitable for performing real-time detection of amplified products in PCR using primer set 1. A probe for a real-time PCR method comprising a polynucleotide which is "nucleotide sequence represented by SEQ ID NO:40" in the "nucleotide sequence represented by SEQ ID NO:38, 39 or 40" of the (2p) is suitable for performing real-time detection of amplified products in PCR using primer set 3.

### <5. Detection method>

The present invention also relates to a method, as defined in claim 9 or claim 10, for detecting M. kansasii and/or a polynucleotide derived from M. kansasii in a sample, the method comprising detecting a polynucleotide in the sample, the polynucleotide comprising a first nucleotide sequence represented by SEQ ID NO:1, 9, 11, 13, 15, 2, 10, 12, 14 or 16; or a second nucleotide sequence complementary to the first nucleotide sequence; or a third nucleotide sequence comprising the first nucleotide sequence or second nucleotide sequence in which any T in is replaced with U.

As described above, since the first nucleotide sequence, the second nucleotide sequence or the third nucleotide sequence are sequences comprised specifically in polynucleotides derived from M. kansasii, it is possible to species-specifically detect M. kansasii and/or polynucleotides derived from M. kansasii by using the sequence as an indicator.

Here, the detection of a polynucleotide comprising the first nucleotide sequence, the second nucleotide sequence or the third nucleotide sequence is not necessary required to detect that the polynucleotide in the sample comprises the entire of the first nucleotide sequence, the second nucleotide sequence or the third nucleotide sequence, and is sufficient to detect that the polynucleotide comprises a characteristic portion of the first nucleotide sequence, the second nucleotide sequence or the third nucleotide sequence.

A sample to be a target in the present invention has a possibility of containing M. kansasii and/or a polynucleotides derived from M. kansasii. Examples of such a sample include a sample containing a part of a living body (organ, tissue, cell, etc.) of animals and plants, feces, body fluid (blood, saliva, urine, sputum, lymph, etc.), food and drink, microbial culture, a PCR product, and the like.

The polynucleotide to be detected may be a naturally occurring polynucleotide or may be an artificially prepared polynucleotide from a naturally occurring polynucleotide. Examples of such a polynucleotide include genomic DNA, mRNA transcribed from the genomic DNA, cDNA prepared from the mRNA, an amplification product obtained by amplifying these polynucleotides, and especially preferably genomic DNA or an amplification product thereof. The terms such as genomic DNA, mRNA and cDNA each encompass a fragment thereof. A polynucleotide to be detected may be present in the form of a double-strand in the sample.

Specific examples of means for detecting a polynucleotide comprising the first nucleotide sequence, the second nucleotide sequence or the third nucleotide sequence in the sample include the method using a primer set of the present invention and the a method using a probe of the present invention. Hereinafter, each embodiment will be described.

### <5.1. Detection Method Using Primer Set of the Present Invention>

One embodiment of the detection method of the present invention comprises
step 1 of performing a nucleic acid amplification reaction on a polynucleotide in a sample as a template by using the primer set of the present invention, and
step 2 of detecting a polynucleotide fragment amplified by the nucleic acid amplification reaction.

The polynucleotide used in step 1 as a template is typically DNA. The polynucleotide may be in a form that has been extracted and purified from the sample, or may be in a form that has been extracted and crude-purified. Alternatively, if the sample contains the polynucleotide at a relatively high concentration, such as a part of cell or tissue, the sample itself may be included as it is in a nucleic acid amplification reaction.

The nucleic acid amplification reaction in step 1 can be carried out according to general PCR methods. In other words, the nucleic acid amplification reaction can be carried out in a PCR condition allowing an amplification of a fragment containing a predetermined target region when performing PCR using the primer set of the present invention in the assumed presence of a polynucleotide comprising the first nucleotide sequence, the second nucleotide sequence or the third nucleotide sequence.

The DNA polymerase used in PCR is not particularly limited as long as it is a heat-resistant DNA polymerase. In the present invention, it is possible to use a commercially available DNA polymerase, and for example, TaKaRa Ex Taq (registered trademark) can be suitably used. The condition such as temperature, time, and composition of the buffer can be appropriately selected depending on DNA polymerase to be used, the concentration of each primer, or the like.

One useful method is a hot start PCR method because it can be expected the effect of suppressing the formation of primer dimers. The hot start PCR method can be performed using a commercially available hot-start PCR reagents, such as TaKaRa Ex Taq (registered trademark) Hot Start Version (Takara Bio). Further, TaKaRa Taq HS PCR Kit, UNG plus (registered trademark) can also be used. By using this kit, it can also be expected the effect of suppressing carryover contamination of nucleic acid amplification products.

The number of cycles in the PCR method is preferably 25 cycles or more, more preferably 30 cycles or more. The upper limit of the number of cycles is not particularly limited, but it is usually 60 cycles or less, preferably 50 cycles or less.

The primer concentration in the reaction mixture at the start of the PCR method is not particularly limited, and it can be appropriately adjusted depending on the polynucleotide to be used as a template or the like. Examples of a preferred primer concentration in the reaction mixture at the beginning of the reaction include a concentration where each of the first primer and second primer is 0.05 µM or more and 1.0 µM or less. If the primer concentration is less than 0.05 µM, there is a possibility that detection sensitivity is reduced, leading to the reduced detection sensitivity basis. The upper limit of the primer concentration is not particularly limited, but it is preferably 1.0 µM.

In step 1, when a sample contains a polynucleotide comprising the first nucleotide sequence, the second nucleotide sequence or the third nucleotide sequence, a polynucleotide fragment comprising a predetermined target region is generated as an amplification product.

In step 2, detection of the polynucleotide fragment amplified by the nucleic acid amplification reaction in step 1 is carried out. Detection in step 2 is not particularly limited, and examples of such a detection include a method by fractioning the reaction solution of the nucleic acid amplification reaction by gel electrophoresis after step 1; and confirming the presence or absence of a band of the size corresponding to the polynucleotide fragment containing a predetermined target region, and a method by hybridizing a reaction solution of nucleic acid amplification reaction or the product thereof with a polynucleotide probe which is a specifically labelled polynucleotide fragment containing a predetermined target region; and detecting the obtained complex.

Further, another embodiment of step 2 includes performing the nucleic acid amplification reaction of step 1 in the presence of a probe of the present invention linked to a reporter fluorescent substance at the 5' end and linked to a quencher dye at the 3' end of the polynucleotide; and detecting a polynucleotide fragment by using the fluorescent, which is generated when a polynucleotide fragments containing the predetermined target region is amplified, as an indicator.

Moreover, when one of the first primer and second primer in the primer set of the present invention further comprises a label portion which is a label substance or a tag capable of binding to a label substance, and the other further comprises a binding portion which is a tag capable of binding to a solid phase carrier, it is possible to perform a following detection step using the solid phase carrier as step 2.

### <5.1.1. Detection Step Using Solid phase carrier>

In this detection step, the product of the nucleic acid amplification reaction in step 1 is contacted with a solid phase carrier containing at least a portion capable of binding to a binding portion, and amplified products at the portion of the solid phase carrier are detected by using the label portion as an indicator.

When the label portion is a labelling tag described above, a labelling step of binding the label substance to the labelling tag may be further performed. Details of the labelling step are described below. When the label portion is a label substance described above, the labelling step is not necessary. The phrase "a label portion as an indicator" in the detection step refers to, when the label portion is a labelling tag, the detection of the amplification product by using a label substance bound through the labelling step as an indicator and, when the label portion is a label substance, the detection of the amplification product by using a label substance as an indicator.

The product of nucleic acid amplification reaction may refer to a reaction solution of nucleic acid amplification reaction possibly containing an amplification product, a sample obtained by further concentrating the amplification product from the reaction solution.

Details of the solid phase carrier are as described above.

Contact of the portion capable of binding to a binding portion in a solid phase carrier with the product can be performed depending on the combination of the solid phase carrier and the binding portion. The contact can be performed in a condition of appropriately adjusted so as to allow the binding between the binding portion of the amplified product contained in the reaction mixture and the portion capable of binding to the binding portion (e.g. aforementioned hybridization conditions or a buffer condition of about pH 5 to 9).

Detection of the amplification products can be carried out by detecting, preferably visually detecting, the label substance bound to the amplification product captured on a solid phase carrier. When the amplified product is present, the label substance of the amplified product captured and bound by the solid phase carrier is detected. By using the presence or absence of the detection as an indicator, it is possible to determine the presence or absence of the amplification product in the reaction mixture of the nucleic acid amplification reaction (a polynucleotide fragment containing a target region) as well as it is possible to determine the presence or absence of M. kansasii and/or a polynucleotide derived from M. kansasii in the sample.

### <5.1.2. Labelling Step>

The labelling step is performed when the label portion included in the primer set of the present invention is a labelling tag described above, and it is performed by contacting the product of the nucleic acid amplification reaction with the label substance, and connecting the labelling tag to the labelling substance. The labelling step may be performed before, after, or during the contact of the product of the nucleic acid amplification reaction with a solid phase carrier.

Contact of the label substance with the product is performed depending on the combination of the label substance and the labelling tag. The contact can be performed in a condition of appropriately adjusted so as to allow the binding between the label substance and the labelling tag of the amplified product (e.g. aforementioned hybridization conditions or a buffer condition of about pH 5 to 9).

### <5.1.3. Detection Device>

The detection step and labelling step described above can be carried out using a nucleic acid detection device utilizing nucleic acid chromatography. By using the nucleic acid detection device, it is possible to detect or determine the presence or absence of an amplification product (polynucleotide containing a target region) in the reaction mixture of a nucleic acid amplification reaction without the need for special equipment, and the result can be obtained easily and rapidly.

The nucleic acid detection device may be a known nucleic acid detection device used to detect the nucleic acid amplification product labeled by a nucleic chromatography method (WO2012/070618).

The schematic diagram of one embodiment of the nucleic acid detection device available in the present invention is shown in Figure 4, but a nucleic acid detection device is not limited to this embodiment. In the following description, reference signs attached to each member correspond to reference signs shown in Figure 4.

The nucleic acid detection device 10 in Figure 4 is formed by disposing, on a substrate 5, a sample pad 3 which is a reaction mixture receiving portion for receiving a reaction mixture of the nucleic acid amplification reaction, a conjugate pad 2 that holds a label substance, a porous solid phase carrier 1 comprising in a part a portion 6 capable of binding to a binding portion contained in the amplification product and an absorbent pad 4 to contact with each other in this order. The portion 6 of the solid phase carrier 1 is a portion where the means for capturing the amplified product (capture means) (e.g., the oligonucleotide described above) is restrictively disposed and immobilized. The surface of the solid phase carrier 1 may be covered by a film, which it not shown. The sample pad 3, the conjugate pad 2, the solid phase carrier 1 and the absorbent pad 4 may be constituted by a member having a porous structure which can be used as the solid phase carrier described above. These may be constituted by the same members, or may be constituted by different members. The substrate 5 is only required that it can support various members disposed thereon and facilitates the manipulation of nucleic acid detection devices. Examples of such a substrate include one made of a resin, a metal, a mineral, or the like. The conjugate pad 2 can be omitted when the label substance is mixed into a development solution or when the label portion is a label substance.

A reaction mixture of the nucleic acid amplification reaction obtained by step 1 is added to the sample pad 3. The reaction mixture may be added as it is, or may be added in a combination with a suitable development solution (e.g., phosphate buffer, Tris buffer, Good's buffer, SSC buffer). If necessary, the development solution may further comprise a surfactant, a salt, a protein, a nucleic acid or the like. The reaction mixture added to the sample pad 3 is developed by a capillary phenomenon from the upstream toward the downstream in the direction indicated by the arrow in Figure 4.

As another embodiment, it can be developed by placing the nucleic acid detection device in the container holding the product and/or development solution of the nucleic acid amplification reaction (e.g., PCR tube, Eppendorf tube, 96 well plate), and immersing the sample pad 3 in a product and/or development solution of the nucleic acid amplification reaction. In that case, so that the sample pad 3 is placed into the container holding the product and/or development solution of the nucleic acid amplification reaction, the width of the sample pad 3 is preferably set to 2.0 to 10.0 mm, more preferably 2.0 to 5.0 mm.

In the embodiment where the label portion is a labelling tag, the amplification product in the reaction mixture contacts with a label substance when passing through the conjugate pad 2 holding a label substance, and labeled with the label substance via a labelling tag.

Then, the amplification product in the reaction mixture contacts with a capture means immobilized to the portion 6 when passing through the solid phase carrier 1, and is captured and bound to the solid phase carrier 1 via a tag for binding.

When the target nucleic acid is present in the sample, the label substance bound to the amplification product captured and bound to the portion 6 of the solid phase carrier 1 comprising capture means is detected at the portion 6. When the label substance is a visually identifiable substance, the portion 6 is colored due to the label substance. By using the presence or absence of detection of the label substance (coloration) as an indicator, it is possible to determine the presence or absence of a target nucleic acid in a sample.

### <5.2. Detection Method Using Probe of the Present Invention>

Another embodiment of the detection method of the present invention comprises
step 3 of incubating a polynucleotide in a sample or a polynucleotide fragment amplified by performing a nucleic acid amplification reaction on a polynucleotide in a sample as a template, with the probe of the present invention under a condition allowing hybridization, and
step 4 of detecting a hybridization of the polynucleotide or the polynucleotide fragment with the probe.

In step 3, the polynucleotide fragment amplified by performing a nucleic acid amplification reaction on the polynucleotide in a sample as a template is preferably an amplification product of the nucleic acid amplification reaction in step 1 above using the primer set of the present invention, but it is not limited to this, and the polynucleotide fragment may be an amplified product of the nucleic acid amplification reaction using another primer set.

In step 3, the "condition allowing hybridization" means a condition in which a so-called specific hybrid is formed and non-specific hybrid is not formed, and it can be appropriately set, for example, with reference to Green and Sambrook, Molecular Cloning, 4th Ed (2012), Cold Spring Harbor Laboratory Press. Specifically, the same condition as "stringent condition" described in <2. Term> with respect to (C) can be exemplified.

In step 4, means for detecting the hybridization of the probe with the polynucleotide or the polynucleotide fragment is not particularly limited.

For example, when the probe of the present invention comprises the label portion, unreacted probes are removed from the reaction mixture after incubation in step 3, and a complex of the polynucleotide or the polynucleotide fragment with the probe can be detected by using the label from the label portion as an indicator. When the label portion is a labelling tag, a labelling step of adding a label substance may be performed as needed.

Further, when the probe of the present invention comprises a binding portion described above, it is possible that a complex of the polynucleotide or the polynucleotide fragment and the probe is immobilized to a solid phase carrier via the binding portion, and then detection of the complex is performed on a solid phase carrier by appropriate means. The probe of the present invention comprising a binding portion may be immobilized on a solid phase carrier via the binding portion in advance.

### <6. Kit>

The present invention also relates to a kit, as defined in claim 6, for detecting Mycobacterium kansasii and/or a polynucleotide derived from Mycobacterium kansasii, the kit comprising the primer set of the present invention and optionally the probe of the present invention.

The kit can be used in the detection method of the present invention.

The kit of the present invention further comprises various reagents for nucleic acid amplification reaction (for example, DNA polymerase, nucleic acid amplification reaction buffer, dNTPs, etc.), various reagents for incubation (incubation buffer), and the like.

When the primer set of the present invention and/or the probe of the present invention comprises a binding portion described above, the kit of the present invention may further comprise a solid phase carrier comprising at least in part a portion capable of binding to the binding portion. As a specific example of the solid phase carrier, the detection device described above is particularly preferred.

When the primer set of the present invention and/or the probe of the present invention comprises a label portion described above and the label portion is a labelling tag, it is preferred that the kit of the present invention further comprises a label substance for labelling the labelling tag.

### Examples

The present invention will be specifically described based on the following experimental results, but the present invention is not limited thereby.

### [Example 1]

### (1) Design of Primers

Primer sets 1 to 3 for PCR amplification detection were designed in the specific region 2 of M. kansasii using Web tools Primer BLAST for primer design. The positions of the primers are as shown in Figures 2-1, 2-2 and 2-3. The nucleotide sequences of forward primer and reverse primer of primer sets 1 to 3 are shown below.

**[Table 3]**

| | Primer | Sequence **(5'→3')** | SEQ ID NO: |
|---|---|---|---|
| Primer set 1 | KAN-1f | TGAATTCCAGGTGGCGTAGG | 3 |
| | KAN-1r | ATCCTGGCAGAAATGCGGAA | 4 |
| Primer set 2 | KAN-2f | CGCAAATCTCGAGTAACCGC | 5 |
| | KAN-2r | CGCTCTTCACCGAATCAACG | 6 |
| Primer set 3 | KAN-3f | TCGTGGACTCCATCCATCGA | 7 |
| | KAN-3r | GGAGCTACGTCCTGTCAGTG | 8 |

### (2) PCR

The PCR reaction solutions below were prepared using primer sets 1 to 3 described above in accordance with the manuals of TaKaRa Taq HS perfect Mix.

**[Table 4]**

| | |
|---|---|
| 5µM Forward primer solution | 1 µL |
| 5µM Reverse primer solution | 1 µL |
| 2x TaKaRa TaqHS perfect Mix | 10 µL |
| Extracted template DNA solution | 1 µL |
| Sterilized water | 7 µL |

As templates, genomic DNAs of M. kansasii type I ATCC12478 strain, M. kansasii type I clinical isolated strain, M. kansasii type VI clinical isolated strain, M. kansasii type II clinical isolated strain, and M. gastri ATCC15754 strain were used. Preparation of genomic DNA from each microorganism strain was carried out in the following procedure: each strain was cultured to 10⁸ cfu/ml or more at Myco Broth (Kyokuto Pharmaceutical Industrial Co., Ltd), and from 10 µl of the obtained cultured solution, DNA was extracted using Kaneka simplified DNA extraction kit ver.2 (Kaneka) in accordance with the protocol of this this product. The obtained solution was used as extracted template DNA solution.

As a negative control, PCR reaction solution having the same composition was prepared except for using 1 µL of sterile distilled water instead of the template DNA solution.

The predetermined primers each were dissolved in sterile distilled water to be a concentration of 5 µM to obtain 5 µM forward primer solution and 5 µM reverse primer solution.

The PCR reaction solutions were set in a thermal cycler (LifeEco, by Bioer Technology Co., LTD.), and PCR was carried out by, after 1 minute reaction at 94°C, repeating 35 cycles of 94°C, 5 seconds/60°C, 10 seconds/72°C, 15 seconds.

### (3) Electrophoresis

Five microliters of the reaction solution obtained in above (2) after PCR was separated by electrophoresis using a 2% agarose gel. Then after stained with ethidium bromide, it was irradiated with ultraviolet rays to detect bands.

The results are shown in Figure 3.

All the primer sets produced the amplification product of target size only when M. kansasii was used as a template. They were able to detect all types of M. kansasii, including type I, type II and type VI. There were no amplified products when M. gastri was used as a template. Consequently, it was confirmed that identification between M. kansasii and M. gastri is possible.

### [Example 2]

Except that genomic DNA from four types of Mycobacterium tuberculosis complex (M. tuberculosis, M. bovis, M. bovis BCG, M. microti) and 38 types of non-tuberculous mycobacteria (M. kansasii, M. gastri. etc.) and one type of general bacteria (Nocardia asteroides) were used as templates, and primer set 2 was used as a primer set, PCR was performed under the same conditions as in Example 1 and the presence or absence of the amplification product was confirmed by an agarose gel electrophoresis method. The results are shown in the following table.

**[Table 5]**

| Microorganism | Strain | Presence or absence of amplified product |
|---|---|---|
| *M. tuberculosis* | ATCC 27294 | Absence |
| *M. bovis* | ATCC 19210 | Absence |
| *M. bovis BCG* | ATCC 35737 | Absence |
| *M. microti* | ATCC 19422 | Absence |
| *M. asiaticum* | ATCC 25276 | Absence |
| *M. avium* | ATCC 25291 | Absence |
| *M. branderi* | ATCC 51789 | Absence |
| *M. celatum* | ATCC 51131 | Absence |
| *M. gastri* | ATCC 15754 | Absence |
| *M. gordonae* | ATCC 14470 | Absence |
| *M. heckeshonense* | DSM 44428 | Absence |
| *M. hiberniae* | DSM 44241 | Absence |
| *M. interjectum* | GTC 689 | Absence |
| *M. intermedium* | ATCC 51848 | Absence |
| *M. intracellulare* | ATCC 13950 | Absence |
| *M*. *kansasii (type I)* | ATCC 12478 | Presence |
| *M. lentifavum* | ATCC 51985 | Absence |
| *M. malmonense* | ATCC 29571 | Absence |
| *M. marinum* | ATCC 927 | Absence |
| *M. nonchromogenicum* | ATCC 19530 | Absence |
| *M. scrofulaceum* | ATCC 19981 | Absence |
| *M. shimoidei* | ATCC 27962 | Absence |
| *M. simiae* | ATCC 25275 | Absence |
| *M. szulgai* | ATCC 35799 | Absence |
| *M. terrae* | ATCC 15755 | Absence |
| *M. triviale* | ATCC 23292 | Absence |
| *M. ulcerans* | ATCC 19423 | Absence |
| *M. xenopi* | ATCC 19520 | Absence |
| *M*. *abscesses* | ATCC 19977 | Absence |
| *M. chelonae* | ATCC 35752 | Absence |
| *M. chitae* | ATCC 19627 | Absence |
| *M. flavescens* | ATCC 11474 | Absence |
| *M. fortuitum* | ATCC 6841 | Absence |
| *M. qilvum* | JCM 6395 | Absence |
| *M. parafortuitum* | JCM 6367 | Absence |
| *M. peregrinum* | ATCC 14467 | Absence |
| *M. phlei* | ATCC 11758 | Absence |
| *M*. *senegalense* | ATCC 35796 | Absence |
| *M. smegmatis* | ATCC 19420 | Absence |
| *M. vaccae* | ATCC 15483 | Absence |
| *M. avium* | ATCC 700898 | Absence |
| *M. qordonae* | GTC 829=KPM 2205 | Absence |
| *N. asteroides* | ATCC 19247 | Absence |

In PCR using primer set 2, amplified products were generated only when genomic DNAs of M. kansasii were used as templates, while there was no PCR amplification when mycobacteria other than M. kansasii and a mycobacteria-closely related general bacteria N. asteroides were used as templates.

From this result, it was demonstrated that the method of the present invention is very effective for species-specific detection of M. kansasii.

### [Example 3]

A nucleic chromatography detection system, which can detect the amplified product by PCR using the primer set 2, was constructed. Using this detection system, it was confirmed that the detection of the clinical isolated strains of M. kansasii is possible.

### (I) Structure of Primers

The 5' end of the forward primer (KAN-2f) of the primer set 2 according to Example 1 was linked to a DNA consisting of the forward primer tag sequences described below via a divalent group represented by formula I containing an azobenzene structure that inhibits polymerase reaction. At this time, the 5' end of the divalent group containing a structure of azobenzene shown by above formula I, and the phosphate group of the 3' end nucleotide of the DNA consisting of forward primer tag sequence form a phosphoester bond; and the phosphate group at the 3' end of the divalent group and the hydroxyl group of position 5' of deoxyribose of the 5' end nucleotide of the primer portion of forward primer form a phosphoric acid ester bond.
5'-GACAACGGAGACAGAGCCAA-3' (SEQ ID NO:41)

The 5' end of the reverse primer (KAN-2r) of the primer set 2 according to Example 1 was linked to a DNA consisting of the reverse primer tag sequence described below via a divalent group represented by formula I above containing an azobenzene structure that inhibits polymerase reaction. At this time, the oxygen atom of one end of the divalent group containing a structure of azobenzene represented by formula I above, and the phosphate group of the 3' end nucleotide of the DNA consisting of reverse primer tag sequence form a phosphoester bond; and the phosphate group at the other end of the divalent group and the hydroxyl group of position 5' of deoxyribose of the 5' end nucleotide of the primer portion of reverse primer form a phosphoric acid ester bond.
5'-ATGCTACCGTATGCCCAGTG-3' (SEQ ID NO:42)

### (II) Preparation of Oligonucleotide Bound Gold Colloid

Gold Colloid (40 nm, 9.0 × 10¹⁰ (number of particles/ml), manufactured by British Biocell International, Inc.) were mixed with a thiol group-containing oligonucleotide represented by SEQ ID NO:43 below, and the mixture was incubated at 50°C for 16 hours. After centrifugation for 15 minutes at 6000 rpm, the supernatant was removed, and 0.05 M sodium chloride and 5 mM phosphate buffer (pH 7) were added and mixed, and then the obtained mixture was incubated again at 50°C for 40 hours.

After incubation, centrifugation (6000 rpm, 15 minutes) was performed, the supernatant was removed, and 5 mM phosphate buffer (pH 7) was added thereto. This buffer replacement was performed again.

The prepared gold colloid solution was added to glass fiber pad to be uniform, and then it was dried in a vacuum dryer to obtain a conjugate pad.
(Thiol Group-Containing Oligonucleotide):
5'-TTGGCTCTGTCTCCGTTGTC-SH-3' (SEQ ID NO:43)

The thiol group-containing oligonucleotide represented by SEQ ID NO:43 is an oligonucleotide in which the phosphate group of position 3' of the cytosine nucleotide at the 3' end is linked to the hydroxyl group of the compound represented by HO-(CH₂)ₘ-SH (wherein m is 6) by a phosphate ester bond.

### (III) Preparation of Membrane Having Immobilized Tag Capture Means

A solution containing an oligonucleotide probe represented by SEQ ID NO:44 below, as a tag capture means, was applied onto a nitrocellulose membrane manufactured by Merck Millipore(Hi-Flowl80) in a line with the width of 1 mm perpendicular to the deployment direction using a dispenser. Then, it was dried at 40°C for 30 minutes to obtain a membrane having a tag capture means.

(Oligonucleotide Probe):
5'-CACTGGGCATACGGTAGCAT-3' (SEQ ID NO:44)

### (IV) Preparation of Lateral Flow Type Nucleic Acid Detection Device

A lateral flow type nucleic acid detection device was prepared in accordance with the detection device shown in the schematic diagram of Figure 4.

Specifically, a polypropylene backing sheet (by Lohmann Technologies) as the substrate 5, a conjugate pad prepared in above (II) as the conjugate pad 2, a membrane (solid phase carrier) 1 having the tag capture means prepared in above (III) as a capture means on the portion 6, a fiberglass sample pad as the sample pad 3, and a cellulosic absorbent pad as the absorbent pad 4 were overlapped and bonded to each other as shown in Figure 4 to prepare a lateral flow type the nucleic acid detection device 10.

### (V) PCR

Using the tag-added primer set 2 described in (I), PCR was performed under the same condition as in Example 1 except for using the genomic DNA of each strain shown in Table 6 as a template.

The type of each strain was determined by a conventional method using a hsp65RPA analysis.

### (VI) Detection 1 using Nucleic Acid Chromatographic Detection System 1

In the reaction solution after PCR under the above described condition, detection of the amplification products was tried using a lateral flow type nucleic acid detection device 10. Specifically, 5 µL of the reaction solution after PCR was added to the sample pad 3 on the device 10, and addition of further 80 µL of a development solution (citrate buffer formulated with a surfactant) was further added to develop the reaction solution. After 10 minutes at room temperature, the presence or absence of colored portion 6 containing a tag capture means immobilized in a line on the membrane 1 was confirmed visually. It was marked "+" when the presence of colored portion was confirmed, and marked "-" when the presence of colored portion was not confirmed.

The results are shown in the following table.

**[Table 6]**

| Sample | | **type** | Determination | Sample | | **type** | Determination | Sample | | type | Determination |
|---|---|---|---|---|---|---|---|---|---|---|---|
| Reference strain | ATCC12478 | I | + | | 31 | I | + | | 62 | I | + |
| | 1 | I | + | | 32 | I | + | | 63 | I | + |
| | 2 | I | + | | 33 | I | + | | 64 | I | + |
| | 3 | I | + | | 34 | I | + | | 65 | I | + |
| | 4 | I | + | | 35 | I | + | | 66 | I | + |
| | 5 | I | + | | 36 | I | + | | 67 | I | + |
| | 6 | I | + | | 37 | I | + | | 68 | I | + |
| | 7 | I | + | | 38 | I | + | | 69 | I | + |
| | 8 | I | + | | 39 | I | + | | 70 | I | + |
| | 9 | I | + | | 140 | I | + | ***M*. *kansasii*** Clinical isolated strain | 71 | I | + |
| | 10 | VI | + | | 41 | I | + | | 72 | I | + |
| | 11 | I | + | | 42 | I | + | | 73 | I | + |
| | 12 | I | + | | 43 | I | + | | 74 | I | + |
| | 13 | I | + | | 44 | I | + | | 75 | II | + |
| | 14 | I | + | | 45 | I | + | | 76 | I | + |
| ***M.kansasii*** Clinical isolated strain | 15 | I | + | ***M. kansasii*** Clinical isolated strain | 46 | I | + | | 77 | I | + |
| | 16 | I | + | | 47 | I | + | | 78 | I | + |
| | 17 | I | + | | 48 | I | + | | 79 | I | + |
| | 18 | I | + | | 49 | I | + | | 80 | I | + |
| | 19 | I | + | | 50 | I | + | | 81 | III | + |
| | 20 | I | + | | 51 | I | + | | | | |
| | 21 | I | + | | 52 | I | + | | | | |
| | 22 | I | + | | 53 | I | + | | | | |
| | 23 | I | + | | 54 | VI | + | | | | |
| | 24 | I | + | | 55 | I | + | | | | |
| | 25 | I | + | | 56 | I | + | | | | |
| | 26 | I | + | | 57 | I | + | | | | |
| | 27 | I | + | | 58 | I | + | | | | |
| | 28 | I | + | | 59 | I | + | | | | |
| | 29 | I | + | | 60 | I | + | | | | |
| | 30 | I | + | | 61 | I | + | | | | |

These results confirmed that all types of M. kansasii, including type I, type II, type III, and type VI, were able to be detected.

Except that genomic DNA extracted from each strain type of M. kansasii shown in Table 6 was used as a template and primer set 2 was used as a primer set, PCR was performed under the same condition as in Example 1 and the presence or absence of the amplification product was confirmed by an agarose gel electrophoresis method. As a result, the presence of an amplification product of the target size could be confirmed in all of the strains.

### (VII) Detection 2 according to the Nucleic Acid Chromatographic Detection System

Except for using the tag-added primer set 2 described in (I) above and using the genomic DNA of each strain shown in Table 5 as a template, PCR was performed under the same conditions as in Example 1.

Then, the reaction solution after the PCR was applied to a lateral flow type nucleic acid detection device 10 with the same procedure as in (VI) above to confirm visually the presence or absence of a colored portion 6 containing a tag capture means immobilized in a line on the membrane 1 in an attempt to detect amplification products.

As a result, coloring was detected (+) only when genomic DNA of M. kansasii (Type I) ATCC12478 was used as a template, and coloring was not detected (-) when the genomic DNA of other strains shown in Table 5 was used as a template.

### [Example 4]

Except that genomic DNA from M. kansasii (Type I) was used as a template and primer set 4 (MKS-40f (SEQ ID NO:22) and MKS-40r (SEQ ID NO:23)), primer set 5 (MKS-41f (SEQ ID NO:24) and MKS-41r (SEQ ID NO:25)), primer set 6 (MKS-42f (SEQ ID NO:26) and MKS-42r (SEQ ID NO:27)) or primer set 7 (MKS-43f (SEQ ID NO:28) and MKS-43r (SEQ ID NO:29)) shown in Table 2 was used as a primer set, PCR was performed under the same condition as in Example 1 and the presence or absence of the amplification product was confirmed by an agarose gel electrophoresis method. All of primer sets could be used to confirm the presence of an amplification product of target size.

### [Example 5]

Except that genomic DNA from M. kansasii (Type II) was used as a template and primer set 6 or primer set 7 shown in Table 2 was used as a primer set, PCR was performed under the same conditions as in Example 1 and the presence or absence of the amplification product was confirmed by an agarose gel electrophoresis method. All of primer sets could be used to confirm the presence of an amplification product of the target size.

### Industrial Applicability

The primer set, probe, method, and kit of the present invention are useful for the accurate detection of Mycobacterium kansasii.

## Claims

1. A primer set which is suitable for species-specific detection of Mycobacterium kansasii comprising:
a first primer comprising a polynucleotide comprising, at the 3' end, a nucleotide sequence A' identical or homologous to a nucleotide sequence A that is a partial nucleotide sequence at the 5' end of a target region in the genomic DNA of Mycobacterium kansasii, the target region comprising all or a part of a first nucleotide sequence represented by SEQ ID NO:1, 9, 11, 13, 15, 2, 10, 12, 14 or 16, or all or a part of a second nucleotide sequence complementary to the first nucleotide sequence; and
a second primer comprising a polynucleotide comprising, at the 3' end, a nucleotide sequence B' identical or homologous to a complementary nucleotide sequence to a nucleotide sequence B that is a partial nucleotide sequence at the 3' end of the target region,
wherein
the first primer comprises
(2f) a polynucleotide having 40 or less bases comprising, at the 3' end, a nucleotide sequence 2fb having consecutive 8 or more bases included in a nucleotide sequence 2fa identical to a nucleotide sequence represented by SEQ ID NO:3, 5 or 7, and
the second primer comprises
(2r) a polynucleotide having 40 or less bases comprising, at the 3' end, a nucleotide sequence 2rb having consecutive 8 or more bases included in a nucleotide sequence 2ra identical to a nucleotide sequence represented by SEQ ID NO:4, 6 or 8; or
wherein
the first primer comprises
(1f) a polynucleotide having 40 or less bases comprising, at the 3' end, a nucleotide sequence 1fb having consecutive 8 or more bases included in a nucleotide sequence If a identical to a nucleotide sequence represented by SEQ ID NO:22, 24, 26, 28, 30, 32, 34 or 36, and
the second primer comprises
(1r) a polynucleotide having 40 or less bases comprising, at the 3' end, a nucleotide sequence 1rb having consecutive 8 or more bases included in a nucleotide sequence 1ra identical to a nucleotide sequence represented by SEQ ID NO:23, 25, 27, 29, 31, 33, 35 or 37.

2. The primer set according to claim 1, wherein one of the first primer and the second primer further comprises a label portion that is a label substance or a tag capable of binding to a label substance; and the other further comprises a binding portion that is a tag capable of binding to a solid phase carrier.

3. A probe comprising
(2p) a polynucleotide comprising a nucleotide sequence 2pb having consecutive 15 or more bases included in a nucleotide sequence 2pa identical to a nucleotide sequence represented by SEQ ID NO:38, 39 or 40 or a complementary nucleotide sequence thereof.

4. The probe according to claim 3, further comprising a label portion that is a label substance or a tag capable of binding to a label substance and/or a binding portion that is a tag capable of binding to a solid phase carrier.

5. The probe according to claim 3 or 4, further comprising a reporter fluorescent dye linked to the 5' end of the polynucleotide, and a quencher dye linked to the 3' end of the polynucleotide.

6. A kit for detecting Mycobacterium kansasii and/or a polynucleotide derived from Mycobacterium kansasii, the kit comprising the primer set according to claims 1 or 2.

7. The kit according to claim 6, comprising a primer set according to claim 2, and further comprising a solid phase carrier comprising at least in part a portion capable of binding to the binding portion.

8. A kit for detecting Mycobacterium kansasii and/or a polynucleotide derived from Mycobacterium kansasii, the kit comprising the probe according to any one of claims 3 to 5.

9. A method for detecting Mycobacterium kansasii and/or a polynucleotide derived from Mycobacterium kansasii in a sample, the method comprising
detecting a polynucleotide in the sample, the polynucleotide comprising a first nucleotide sequence represented by SEQ ID NO:1, 9, 11, 13, 15, 2, 10, 12, 14 or 16; a second nucleotide sequence complementary to the first nucleotide sequence; or a third nucleotide sequence comprising the first nucleotide sequence or second nucleotide sequence in which any T is replaced with U,
wherein the detecting a polynucleotide comprises
performing a nucleic acid amplification reaction on the polynucleotide in the sample as a template by using the primer set according to claims 1 or 2, and
detecting a polynucleotide fragment amplified by the nucleic acid amplification reaction.

10. A method for detecting Mycobacterium kansasii and/or a polynucleotide derived from Mycobacterium kansasii in a sample, the method comprising
detecting a polynucleotide in the sample, the polynucleotide comprising a first nucleotide sequence represented by SEQ ID NO:1, 9, 11, 13, 15, 2, 10, 12, 14 or 16; a second nucleotide sequence complementary to the first nucleotide sequence; or a third nucleotide sequence comprising the first nucleotide sequence or second nucleotide sequence in which any T is replaced with U,
wherein the detecting a polynucleotide comprises
incubating the polynucleotide in the sample or a polynucleotide fragment amplified by performing a nucleic acid amplification reaction on the polynucleotide in a sample as a template, with the probe according to any one of claims 3 to 5 under a condition allowing hybridization, and
detecting the hybridization of the polynucleotide or the polynucleotide fragment with the probe.

## Patentansprüche

1. Primer-Set, das für den Spezies-spezifischen Nachweis von *Mycobacterium kansasii* geeignet ist, umfassend:
einen ersten Primer, der ein Polynucleotid umfasst, das am 3'-Ende eine Nucleotidsequenz A' umfasst, die identisch mit oder homolog zu einer Nucleotidsequenz A ist, die eine Teil-Nucleotidsequenz am 5'-Ende einer Zielregion in der genomischen DNA von *Mycobaterium kansasii* ist, wobei die Zielregion die gesamte oder einen Teil einer ersten Nucleotidsequenz umfasst, die von SEQ ID NO:1, 9, 11, 13, 15, 2, 10, 12, 14 oder 16 dargestellt ist, oder die gesamte oder einen Teil einer zweiten Nucleotidsequenz umfasst, die zu der ersten Nucleotidsequenz komplementär ist; und
einen zweiten Primer, der ein Polynucleotid umfasst, das am 3'-Ende eine Nucleotidsequenz B' umfasst, die identisch mit oder homolog zu einer Nucleotidsequenz ist, die zu einer Nucleotidsequenz B komplementär ist, die eine Teil-Nucleotidsequenz am 3'-Ende der Zielregion ist,
wobei
der erste Primer
(2f) ein Polynucleotid mit 40 oder weniger Basen umfasst, das am 3'-Ende eine Nucleotidsequenz 2fb mit aufeinanderfolgenden 8 oder mehr Basen umfasst, die in einer Nucleotidsequenz 2fa enthalten sind, die mit einer durch SEQ ID NO:3, 5 oder 7 dargestellten Nucleotidsequenz identisch ist; und
der zweite Primer
(2r) ein Polynucleotid mit 40 oder weniger Basen umfasst, das am 3'-Ende eine Nucleotidsequenz 2rb mit aufeinanderfolgenden 8 oder mehr Basen umfasst, die in einer Nucleotidsequenz 2ra enthalten sind, die mit einer durch SEQ ID NO:4, 6 oder 8 dargestellten Nucleotidsequenz identisch ist; oder
wobei
der erste Primer
(1f) ein Polynucleotid mit 40 oder weniger Basen umfasst, das am 3'-Ende eine Nucleotidsequenz 1fb mit aufeinanderfolgenden 8 oder mehr Basen umfasst, die in einer Nucleotidsequenz 1fa enthalten sind, die mit einer durch SEQ ID NO:22, 24, 26, 28, 30, 32, 34 oder 36 dargestellten Nucleotidsequenz identisch ist; und
der zweite Primer
(1r) ein Polynucleotid mit 40 oder weniger Basen umfasst, das am 3'-Ende eine Nucleotidsequenz 1rb mit aufeinanderfolgenden 8 oder mehr Basen umfasst, die in einer Nucleotidsequenz 1ra enthalten sind, die mit einer durch SEQ ID NO:23, 25, 27, 29, 31, 33, 35 oder 37 dargestellten Nucleotidsequenz identisch ist.

2. Primer-Set nach Anspruch 1, wobei einer aus dem ersten Primer und dem zweiten Primer des Weiteren einen Markerteil umfasst, der eine Markersubstanz oder ein Tag ist, die/das fähig ist, an eine Markersubstanz zu binden, und der andere des Weiteren einen Bindungsteil umfasst, das ein Tag ist, das fähig ist, an einen Festphasenträger zu binden.

3. Sonde, die
(2p) ein Polynucleotid umfasst, das eine Nucleotidsequenz 2pb mit aufeinanderfolgenden 15 oder mehr Basen umfasst, die in einer Nucleotidsequenz 2pa enthalten sind, die mit einer durch SEQ ID NO:38, 39 oder 40 dargestellten Nucleotidsequenz oder einer komplementären Nucleotidsequenz davon identisch ist.

4. Sonde nach Anspruch 3, die des Weiteren einen Markerteil umfasst, der eine Markersubstanz oder ein Tag ist, die/das fähig ist, an eine Markersubstanz zu binden und/oder einen Bindungsteil umfasst, der ein Tag ist, das fähig ist, an einen Festphasenträger zu binden.

5. Sonde nach Anspruch 3 oder 4, die des Weiteren einen Reporter-Fluoreszenzfarbstoff, der an das 5'-Ende des Polynucleotids gebunden ist, und einen Quencher-Farbstoff, der an das 3'-Ende des Polynucleotids gebunden ist, umfasst.

6. Kit zum Nachweis von *Mycobacterium kansasii* und/oder von einem Polynucleotid, das von *Mycobacterium kansasii* stammt, wobei der Kit das Primer-Set nach Anspruch 1 oder 2 umfasst.

7. Kit nach Anspruch 6, der ein Primer-Set nach Anspruch 2 umfasst und der des Weiteren einen Festphasenträger umfasst, der mindestens teilweise einen Teil umfasst, derfähig ist, an den Bindungsteil zu binden.

8. Kit zum Nachweis von *Mycobacterium kansasii* und/oder von einem Polynucleotid, das von *Mycobacterium kansasii* stammt, wobei der Kit die Sonde nach einem der Ansprüche 3 bis 5 umfasst.

9. Verfahren zum Nachweis von *Mycobacterium kansasii* und/oder von einem Polynucleotid, das von *Mycobacterium kansasii* stammt, in einer Probe, wobei das Verfahren umfasst:
das Nachweisen eines Polynucleotids in der Probe, wobei das Polynucleotid eine erste Nucleotidsequenz umfasst, die durch SEQ ID NO:1, 9, 11, 13, 15, 2, 10, 12, 14 oder 16 dargestellt ist; eine zweite Nucleotidsequenz, die zu der ersten Nucleotidsequenz komplementär ist; oder eine dritte Nucleotidsequenz, die die erste Nucleotidsequenz oder die zweite Nucleotidsequenz, in der ein beliebiges T mit U ersetzt ist, umfasst,
wobei das Nachweisen eines Polynucleotids umfasst:
das Durchführen einer Nucleinsäureamplifikationsreaktion auf dem Polynucleotid in der Probe als Matrize durch die Verwendung des Primer-Sets nach Anspruch 1 oder 2 und
das Nachweisen eines Polynucleotidfragments, das durch die Nucleinsäureamplifikationsreaktion amplifiziert wird.

10. Verfahren zum Nachweis von *Mycobacterium kansasii* und/oder von einem Polynucleotid, das von *Mycobacterium kansasii* stammt, in einer Probe, wobei das Verfahren das Nachweisen eines Polynucleotids in der Probe umfasst, wobei das Polynucleotid eine erste Nucleotidsequenz umfasst, die durch SEQ ID NO:1, 9, 11, 13, 15, 2, 10, 12, 14 oder 16 dargestellt ist; eine zweite Nucleotidsequenz, die zu der ersten Nucleotidsequenz komplementär ist; oder eine dritte Nucleotidsequenz, die die erste Nucleotidsequenz oder die zweite Nucleotidsequenz, in der ein beliebiges T mit U ersetzt ist, umfasst,
wobei das Nachweisen eines Polynucleotids umfasst:
das Inkubieren des Polynucleotids in der Probe oder eines Polynucleotidfragments, das durch das Durchführen einer Nucleinsäureamplifikationsreaktion auf dem Polynucleotid in einer Probe als Matrize amplifiziert wurde, mit der Sonde nach einem der Ansprüche 3 bis 5 unter einer Bedingung, die eine Hybridisierung erlaubt, und
das Nachweisen der Hybridisierung des Polynucleotids oder des Polynucleotidfragments mit der Sonde.

## Revendications

1. Ensemble d'amorces qui est approprié pour la détection spécifique d'espèce de Mycobacterium kansasii comprenant:
une première amorce comprenant un polynucléotide comprenant, à l'extrémité 3', une séquence nucléotidique A' identique à ou homologue d'une séquence nucléotidique A qui est une séquence nucléotidique partielle à l'extrémité 5' d'une région cible dans l'ADN génomique de Mycobacterium kansasii, la région cible comprenant tout ou partie d'une première séquence nucléotidique représentée par SEQ ID NO: 1, 9, 11, 13, 15, 2, 10, 12, 14 ou 16, ou tout ou partie d'une seconde séquence nucléotidique complémentaire de la première séquence nucléotidique; et
une seconde amorce comprenant un polynucléotide comprenant, à l'extrémité 3', une séquence nucléotidique B' identique à ou homologue d'une séquence nucléotidique complémentaire à une séquence nucléotidique B qui est une séquence nucléotidique partielle à l'extrémité 3' de la région cible, où
la première amorce comprend
(2f) un polynucléotide ayant 40 bases ou moins comprenant, à l'extrémité 3', une séquence nucléotidique 2fb ayant 8 bases consécutives ou plus incluse dans une séquence nucléotidique 2fa identique à une séquence nucléotidique représentée par SEQ ID NO: 3, 5 ou 7, et
la seconde amorce comprend
(2r) un polynucléotide ayant 40 bases ou moins comprenant, à l'extrémité 3', une séquence nucléotidique 2rb ayant 8 bases consécutives ou plus incluse dans une séquence nucléotidique 2ra identique à une séquence nucléotidique représentée par SEQ ID NO: 4, 6 ou 8; ou
où
la première amorce comprend
(1f) un polynucléotide ayant 40 bases ou moins comprenant, à l'extrémité 3', une séquence nucléotidique 1fb ayant 8 bases consécutives ou plus incluse dans une séquence nucléotidique 1fa identique à une séquence nucléotidique représentée par SEQ ID NO: 22, 24, 26, 28, 30, 32, 34 ou 36, et
la seconde amorce comprend
(1r) un polynucléotide ayant 40 bases ou moins comprenant, à l'extrémité 3', une séquence nucléotidique 1rb ayant 8 bases consécutives ou plus incluse dans une séquence nucléotidique 1ra identique à une séquence nucléotidique représentée par SEQ ID NO: 23, 25, 27, 29, 31, 33, 35 ou 37.

2. Ensemble d'amorces selon la revendication 1, où l'une de la première amorce et de la seconde amorce comprend en outre une partie marqueur qui est une substance marqueur ou une étiquette capable de se lier à une substance marqueur; et l'autre comprend en outre une partie de liaison qui est une étiquette capable de se lier à un support en phase solide.

3. Sonde comprenant
(2p) un polynucléotide comprenant une séquence nucléotidique 2pb ayant 15 bases consécutives ou plus incluse dans une séquence nucléotidique 2pa identique à une séquence nucléotidique représentée par SEQ ID NO: 38, 39 ou 40 ou une séquence nucléotidique complémentaire de celle-ci.

4. Sonde selon la revendication 3, comprenant en outre une partie marqueur qui est une substance marqueur ou une étiquette capable de se lier à une substance marqueur et/ou une partie de liaison qui est une étiquette capable de se lier à un support en phase solide.

5. Sonde selon la revendication 3 ou 4, comprenant en outre un colorant fluorescent rapporteur lié à l'extrémité 5' du polynucléotide, et un colorant extincteur lié à l'extrémité 3' du polynucléotide.

6. Kit pour détecter Mycobacterium kansasii et/ou un polynucléotide dérivé de Mycobacterium kansasii, le kit comprenant l'ensemble d'amorces selon les revendications 1 ou 2.

7. Kit selon la revendication 6, comprenant un ensemble d'amorces selon la revendication 2, et comprenant en outre un support en phase solide comprenant au moins en partie une partie capable de se lier à la partie de liaison.

8. Kit pour détecter Mycobacterium kansasii et/ou un polynucléotide dérivé de Mycobacterium kansasii, le kit comprenant la sonde selon l'une quelconque des revendications 3 à 5.

9. Procédé pour détecter Mycobacterium kansasii et/ou un polynucléotide dérivé de Mycobacterium kansasii dans un échantillon, le procédé comprenant
la détection d'un polynucléotide dans l'échantillon, le polynucléotide comprenant une première séquence nucléotidique représentée par SEQ ID NO: 1, 9, 11, 13, 15, 2, 10, 12, 14 ou 16; une seconde séquence nucléotidique complémentaire de la première séquence nucléotidique; ou une troisième séquence nucléotidique comprenant la première séquence nucléotidique ou la seconde séquence nucléotidique dans laquelle tout T est remplacé par U,
où la détection d'un polynucléotide comprend
la conduite d'une réaction d'amplification d'acide nucléique sur le polynucléotide dans l'échantillon en tant que matrice en utilisant l'ensemble d'amorces selon les revendications 1 ou 2, et
la détection d'un fragment de polynucléotide amplifié par la réaction d'amplification d'acide nucléique.

10. Procédé pour détecter Mycobacterium kansasii et/ou un polynucléotide dérivé de Mycobacterium kansasii dans un échantillon, le procédé comprenant
la détection d'un polynucléotide dans l'échantillon, le polynucléotide comprenant une première séquence nucléotidique représentée par SEQ ID NO: 1, 9, 11, 13, 15, 2, 10, 12, 14 ou 16; une seconde séquence nucléotidique complémentaire de la première séquence nucléotidique; ou une troisième séquence nucléotidique comprenant la première séquence nucléotidique ou la seconde séquence nucléotidique dans laquelle tout T est remplacé par U,
où la détection d'un polynucléotide comprend
l'incubation du polynucléotide dans l'échantillon ou un fragment de polynucléotide amplifié par la conduite d'une réaction d'amplification d'acide nucléique sur le polynucléotide dans un échantillon en tant que matrice, avec la sonde selon l'une quelconque des revendications 3 à 5 dans des conditions permettant l'hybridation, et
la détection de l'hybridation du polynucléotide ou du fragment de polynucléotide avec la sonde.
